(19) Europäisches Patentamt
European Patent Office
Office européen des brevets

(11) EP 3 012 247 B1

(12) **EUROPEAN PATENT SPECIFICATION**

(45) Date of publication and mention of the grant of the patent:
**10.01.2018 Bulletin 2018/02**

(21) Application number: **14813379.6**

(22) Date of filing: **16.06.2014**

(51) Int Cl.:
***C07C 255/64*** *(2006.01)* ***C07C 259/14*** *(2006.01)*
***C07B 61/00*** *(2006.01)*

(86) International application number:
**PCT/JP2014/065891**

(87) International publication number:
**WO 2014/203855 (24.12.2014 Gazette 2014/52)**

# (54) MANUFACTURING METHOD FOR 2-AMINO-2-HYDROXYIMINO-N-ALKOXY ACETOIMIDOYL CYANIDE, AND MANUFACTURING INTERMEDIATE THEREOF

HERSTELLUNGSVERFAHREN FÜR 2-AMINO-2-HYDROXYIMINO-N-ALKOXY-ACETOIMIDOYLCYANID UND HERSTELLUNG EINES ZWISCHENPRODUKTS DAVON

PROCÉDÉ DE PRODUCTION D'UN CYANURE DE 2-AMINO-2-HYDROXYIMINO-N-ALCOXY ACÉTOIMIDOYLE, ET INTERMÉDIAIRE POUR PRODUIRE LEDIT COMPOSÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2013 JP 2013130079**

(43) Date of publication of application:
**27.04.2016 Bulletin 2016/17**

(73) Proprietor: **Kumiai Chemical Industry Co., Ltd.**
**Tokyo 110-0008 (JP)**

(72) Inventors:
- **YASUMURA Shingo**
**Fuji-shi**
**Shizuoka 421-3306 (JP)**
- **HIRANO Yuuki**
**Fuji-shi**
**Shizuoka 421-3306 (JP)**

(74) Representative: **Keller, Günter et al**
**Lederer & Keller**
**Patentanwälte Partnerschaft mbB**
**Unsöldstrasse 2**
**80538 München (DE)**

(56) References cited:
**WO-A1-2013/080479**
**JP-A- H0 477 477**
**JP-A- H04 187 667**
**JP-A- S57 158 769**
**US-A- 3 234 266**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 012 247 B1

## Description

Technical Field

The present invention relates to a 2-alkoxyiminopropanedinitrile of the general formula (2) :

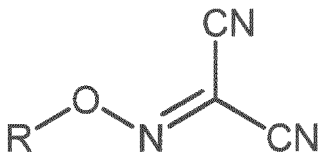

(2)

wherein R is an alkyl group having 3 to 6 carbon atoms, and a process for the production thereof. The 2-alkoxyiminopropanedinitrile of the general formula (2) is a useful intermediate for producing the 2-amino-2-hydroxyimino-N-alkoxyacetimidoylcyanide of the general formula (3), in the process of the present invention.

This invention also relates to a process for the production of a 2-amino-2-hydroxyimino-N-alkoxyacetimidoylcyanide of the general formula (3):

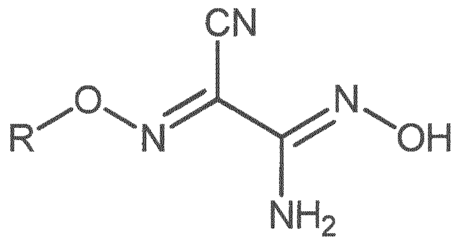

(3)

wherein R is as defined above. The 2-amino-2-hydroxyimino-N-alkoxyacetimidoylcyanide of the general formula (3) is useful as an agricultural chemical by itself, and is also useful as an intermediate for the production of another agricultural chemical.

Background Art

A 2-amino-2-hydroxyimino-N-alkoxyacetimidoylcyanide of the general formula (3) is a useful compound having excellent pesticidal activity (see Patent Literature 1). For example, specifically, 2-amino-2-hydroxyimino-N-isopropoxyacetimidoylcyanide exhibits excellent pesticidal activity.

In addition, as shown in the following scheme, a 2-amino-2-alkoxyimino-N-alkoxyacetimidoylcyanide of the general formula (5) can be produced from a 2-amino-2-hydroxyimino-N-alkoxyacetimidoylcyanide of the general formula (3).

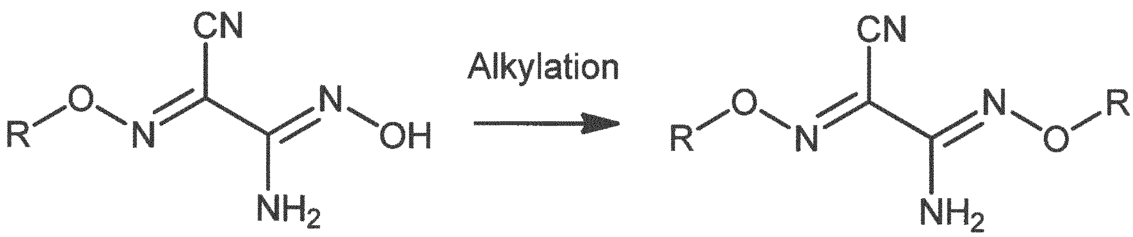


(3) (5)

For example, as specifically described in Example 1 (2) and Example 2 of the Patent Literature 1, a 2-amino-2-alkoxyimino-N-alkoxyacetimidoylcyanide of the general formula (5) can be produced by alkylation of a 2-amino-2-hydroxyimino-N-alkoxyacetimidoylcyanide of the general formula (3).

Furthermore, the Patent Literature 1 discloses that a 2-amino-2-alkoxyimino-N-alkoxyacetimidoylcyanide of the general formula (5) also has excellent pesticidal activity.

For example, specifically, 2-amino-2-isopropoxyimino-N-isopropoxyacetimidoylcyanide, 2-amino-2-isopropoxyimino-N-propoxyacetimidoylcyanide and 2-amino-2-propoxyimino-N-propoxyacetimidoylcyanide exhibit excellent pesticidal activity.

On the other hand, the 2-amino-2-alkoxyimino-N-alkoxyacetimidoylcyanide of general formula (5) can be used as an intermediate for producing an alkoxyimino derivative disclosed in the Patent Literature 2. In other words, as shown in the following scheme, some of alkoxyimino derivatives, which are disclosed in the Patent Literature 2, can be produced from 2-amino-2-alkoxyimino-N-alkoxyacetimidoylcyanide of formula (5) by the method known to the skilled person.

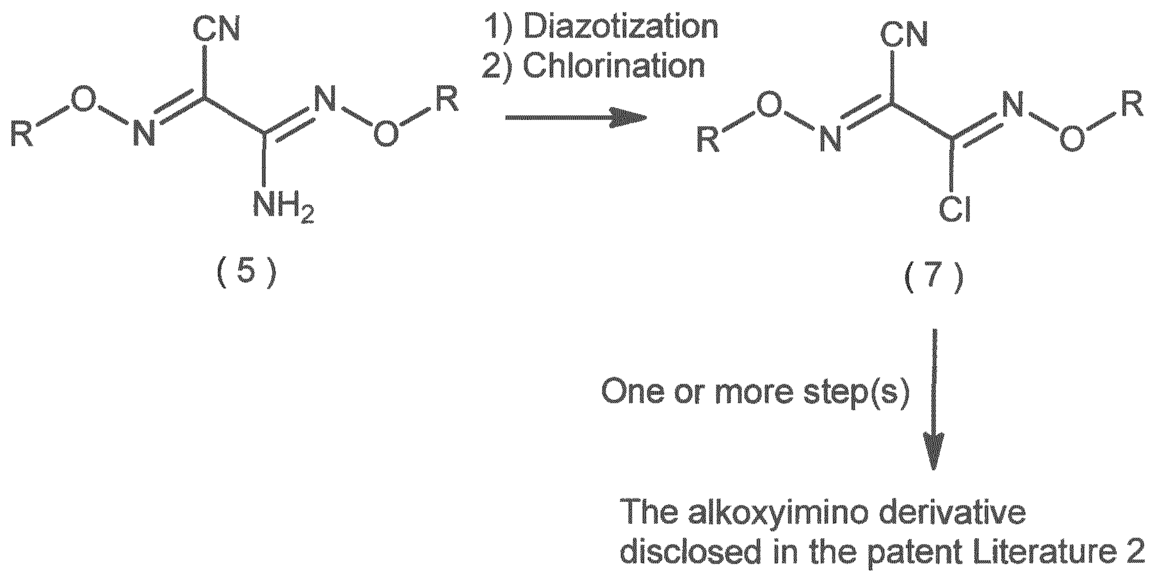


(5)

(7)

For example, the target compound of Example 1 (2) of Patent Literature 2 can be produced from the above 2-amino-2-isopropoxyimino-N-isopropoxyacetimidoylcyanide, in the similar manner as Example 1 of Patent Literature 3, consisting of diazotization followed by chlorination. For another example, the target compound of Example 23 (4) of Patent Literature 2 can be produced from the above 2-amino-2-propoxyimino-N-propoxyacetimidoylcyanide, in the same manner.

And, the target compound of Example 1 (2) of Patent Literature 2 is an intermediate for producing a variety of alkoxyimino derivatives, as specifically described in other Examples of the Patent Literature 2. Also, the target compound of Example 23 (4) of Patent Literature 2 is an intermediate for producing some of alkoxyimino derivatives, as specifically described in other Examples of the Patent Literature 2.

In this context, an alkoxyimino derivative, which is disclosed in the Patent Literature 2, is also known as a useful compound having excellent pesticidal activity. As disclosed in the Patent Literature 2, the pest control agent containing an alkoxyimino derivative as an active ingredient shows an excellent control effect to a wide variety of pests in agricultural and horticultural fields, and is highly effective particularly to Hemipteran pests such as Nilaparvata lugens (brown rice planthopper), Aphis gossypii (aphid), and the like. Additionally, the pest control agent containing an alkoxyimino derivative as an active ingredient can control even resistant pests. Specific examples of the alkoxyimino derivative, which has been produced from above-mentioned the target compound of Example 1 (2) or Example 23 (4) of Patent Literature 2 and exhibit excellent pesticidal activity, include 1-(2-cyano-1,2-diisopropoxyiminoethyl)-1H-1,2,4-triazole, 1-(2-carbamoyl-1,2-diisopropoxyiminoethyl)-1H-1,2,4-triazole, 1-(2-cyano-l(2-dipropoxyiminoethyl)-1H-1,2,4-triazole, 1-(2-carbamoyl-1,2-dipropoxyiminoethyl)-1H-1,2,4-triazole and the like,
preferably 1-(2-carbamoyl-1,2-diisopropoxyiminoethyl)-1H-1,2,4-triazole, 1-(2-carbamoyl-1,2-dipropoxyiminoethyl)-1H-1,2,4-triazole, particularly preferably 1-(2-carbamoyl-1,2-diisopropoxyiminoethyl)-1H-1,2,4-triazole.

In short, a 2-amino-2-hydroxyimino-N-alkoxyacetimidoylcyanide of the general formula (3) is not only useful as an active ingredient of a pest control agent by itself, but also useful as an intermediate for producing the series of the active ingredients of a pest control agents, disclosed in the Patent Literature 1 and 2.

Conventionally, as shown in the following scheme, a process using 2-amino-2-hydroxyimino-N-hydroxyacetimidoylcyanide of the formula (6) as an intermediate is known as a process for producing a 2-amino-2-hydroxyimino-N-alkoxyacetimidoylcyanide of the general formula (3) (see Patent Literature 1).

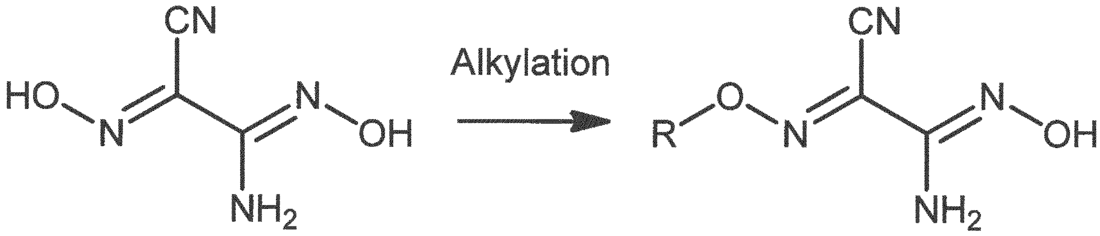


( 6 ) ( 3 )

However, Non-Patent Literature 1 suggests that the differential scanning calorimetric analysis of 2-amino-2-hydroxyimino-N-hydroxyacetimidoylcyanide of the formula (6) showed its highly dangerous thermal behavior, i.e., exothermic decomposition reaction. In fact, Non-Patent Literature 1 specifically discloses that the monohydrate of 2-amino-2-hydroxyimino-N-hydroxyacetimidoylcyanide of the formula (6) exhibits exothermic onset temperature of 124°C and the amount of heat released of 10.3 kJ/g. These values are extremely risky, and 2-amino-2-hydroxyimino-N-hydroxyacetimidoylcyanide of the formula (6) is a dangerous compound. The calorific value as large as 10.3 kJ/g suggests that decomposition reaction is so high exothermic that it leads to an uncontrollable condition in a reactor. In addition, the low onset temperature of 124°C means that the said exothermic decomposition reaction starts at relatively low temperature. Therefore, this process cannot ensure the safety of the production plant, because there is a possibility that a runaway of a reaction and an explosion can occur. That is, this process using 2-amino-2-hydroxyimino-N-hydroxyacetimidoylcyanide of the formula (6) is not preferred for industrial manufacture, because of lack of safety.

In the situation described above, the present inventors had studied a process for the production of a 2-amino-2-hydroxyimino-N-alkoxyacetimidoylcyanide of the general formula (3), which process do not use the dangerous compound such as 2-amino-2-hydroxyimino-N-hydroxyacetimidoylcyanide of the formula (6). As a result, the present inventors succeeded in producing a novel 2-alkoxyiminopropanedinitrile of the following the general formula (2), wherein R is an alkyl group having 3 to 6 carbon atoms.

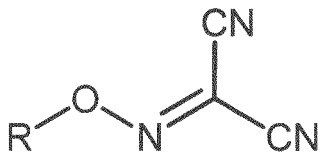

( 2 )

Furthermore, as shown in the following scheme, the present inventors unexpectedly found that a 2-amino-2-hydroxyimino-N-alkoxyacetimidoylcyanide of the general formula (3) can be produced safely from the 2-alkoxyiminopropanedinitrile of the general formula (2), wherein any R is as defined above.

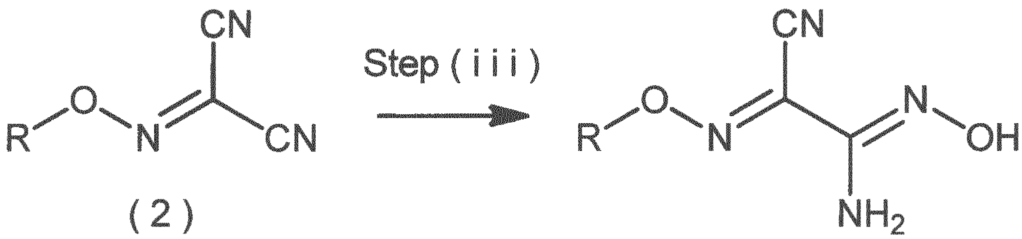


( 2 )

( 3 )

Meanwhile, the process for producing some of analogues of the said 2-alkoxyiminopropanedinitrile is known. As a process for producing the analogues of the said 2-alkoxyiminopropanedinitrile, a process of reacting malononitrile with an alkali metal salt of nitrous acid, and then reacting the resulting compound with an alkylating agent, has heretofore been known.

However, in this conventionally known process, alkylating agents which can achieve a high yield, are limited to alkylating agents having high reactivity, such as dimethyl sulfate, α-halocarboxylic acid esters and benzyl bromide (see Patent Literature 4, 5, 6 and 7, and Non-Patent Literature 2). In fact, it has been reported that the yield is as poor as 24%, when an alkylating agent having low reactivity such as 2-bromofluoro-1,1-ethane is used, even though the reaction is carried out in the N,N-dimethylformamide as solvent (see Patent Literature 8).

In addition, Patent Literature 9 discloses that the reaction of the silver salt of 2-hydroxyiminopropanedinitrile with cyclopropylmethylbromide achieved 64% of yield. However this process is not industrially preferable because the silver salt is expensive.

In short, by the present inventors, the possibility is found that a 2-alkoxyiminopropanedinitrile of the general formula (2) would be a useful intermediate in the process of the present invention. However, an industrially satisfactory process for production of the 2-alkoxyiminopropanedinitrile of the general formula (2) has not been known.

Citation List

Patent Literature

Patent Literature 1: WO 2013/080479 A1 (published on June 6, 2013)
Patent Literature 2: WO 2011/161945 A1
Patent Literature 3: US 2006/0258719 A1
Patent Literature 4: Japanese Patent Application Laid-Open No. 4-187667 (JP-A-1992-187667)
Patent Literature 5: EP 0,397,511 B1
Patent Literature 6: Japanese Patent Laid-Open No. 6-4647 (JP-B-1994-004647)
Patent Literature 7: EP 0,069,872 B1
Patent Literature 8: EP 0,517,041 A1
Patent Literature 9: EP 0,150,609 A2

Non-Patent Literature

Non-Patent Literature 1: J. Org. Chem., 2000, 65, 1139-1143
Non-Patent Literature 2: Bull. Chem. Soc. Jpn., 76, 1063-1070 (2003)

Compounds which are structurally comparable to the compound of general formula (2) are known from JP H04 187667, US 3,234,266, JP H04 77477 and JP S57 158769. The difference can be seen, however, in the definition of substituent R which is according to general formula (2) a C3 to C6 alkyl group. The structurally similar compounds as disclosed in those publications are also used as intermediates, however, not for the synthesis of compounds of the general formula (3), but for the synthesis of other heterocyclic compounds.

WO 2013/080479 relates to the synthesis of pesticides of formula (I). The synthetic pathway leading to the compounds of general formula (3) differs, however, from the synthetic procedures disclosed in WO 2013/080479.

Summary of Invention

Technical Problem

An object of the present invention is to provide a process for the production of a 2-amino-2-hydroxyimino-N-alkoxyacetimidoylcyanide of the general formula (3), which process is suitable for industrialization and economically preferable.

Specifically, for example, the object of the present invention is to provide a safer process for the production of a 2-amino-2-hydroxyimino-N-alkoxyacetimidoylcyanide of the general formula (3), which process does not use the thermally dangerous compound such as 2-amino-2-hydroxyimino-N-hydroxyacetimidoylcyanide of the formula (6), as an intermediate for the production.

In order to achieve the object of the above a safer process, there is a need to provide a safer intermediate for the production.

In addition, there is also a need to provide an industrially preferred process for producing such a safer intermediate for the production. In other words, as described herein, a 2-alkoxyiminopropanedinitrile of the general formula (2) is provided for such a safer intermediate for the production, however there is a need to provide an industrially satisfactory process for production thereof. That is, another object of the present invention is to provide a process for the production of the 2-alkoxyiminopropanedinitrile of the general formula (2), which process is industrially preferable. For example, there is a need to provide a process in which 2-alkoxyiminopropanedinitriles can be produced with a high yield, even when the above-mentioned alkylating agent having high reactivity is not used. Furthermore, a process for

the production thereof without using an expensive raw material and so on, such as the silver salt, has been desired.

Solution to Problem

In view of the circumstances as described above, the present inventors have earnestly studied a process for the production of a 2-amino-2-hydroxyimino-N-alkoxyacetimidoylcyanide of the general formula (3). And as a result, the present inventors unexpectedly found that the above problems can be solved by using a novel compound, i.e., 2-alkoxyiminopropanedinitrile of the general formula (2):

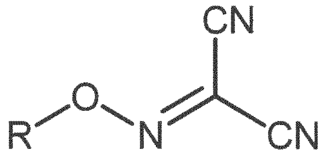

(2)

wherein R is C3 to C6 alkyl group,
and by simultaneously providing a novel process for the production thereof, which process is industrially satisfactory. In other words, the present inventors unexpectedly found that a compound, i.e., 2-amino-2-hydroxyimino-N-alkoxyacetimidoylcyanide of the general formula (3):

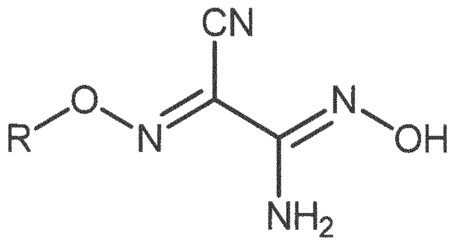

(3)

wherein R is C3 to C6 alkyl group,
can be safely produced with a high yield,
by reacting malononitrile of the formula (1):

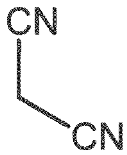

(1)

with an alkali metal salt of nitrous acid in the presence of a protic acid,
followed by reacting the resulting product with C3 to C6 alkyl halide in the presence of a phase transfer catalyst,
and then reacting the resulting compound, i.e., 2-alkoxyiminopropanedinitrile of the general formula (2):

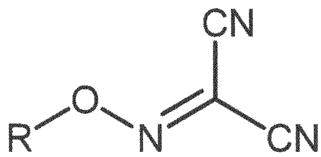

(2)

wherein R is as defined above,
with a hydroxylamine compound. Based on these findings, the present inventors have completed the present invention.

Advantageous Effects of Invention

The present invention provides a novel industrially applicable production process for a 2-amino-2-hydroxyimino-N-alkoxyacetimidoylcyanide of the general formula (3).

Simultaneously, the present invention provides a novel 2-alkoxyiminopropanedinitrile of the general formula (2) and an industrially satisfactory production process for same.

According to the present invention, the target 2-amino-2-hydroxyimino-N-alkoxyacetimidoylcyanide of the general formula (3) can be produced, avoiding the use of a thermally dangerous compound as an intermediate for the production thereof. In this context, as described above, a dangerous production intermediate in the prior art is 2-amino-2-hydroxyimino-N-hydroxyacetimidoylcyanide of the formula (6). That is, according to the present invention, the target 2-amino-2-hydroxyimino-N-alkoxyacetimidoylcyanide of the general formula (3) can be produced, without using 2-amino-2-hydroxyimino-N-hydroxyacetimidoylcyanide of the formula (6), even monohydrate of which exhibits an exothermic decomposition with an exothermic onset temperature of 124°C and the amount of heat released of 10.3 kJ/g on differential scanning calorimetry.

Meanwhile, a production intermediate used in the process of the present invention is a 2-alkoxyiminopropanedinitrile of the general formula (2). Specific example of a 2-alkoxyiminopropanedinitrile of the general formula (2) includes 2-isopropoxyiminopropanedinitrile. The differential scanning calorimetric measurement of 2-isopropoxyiminopropanedinitrile gave exothermic onset temperature of 225°C and the amount of heat released of 0.6 kJ/g. The exothermic onset temperature of 2-isopropoxyiminopropanedinitrile is remarkably higher than the one of 2-amino-2-hydroxyimino-N-hydroxyacetimidoylcyanide of the formula (6), which is the production intermediate in the prior art. This exothermic onset temperature means far better thermal stability than the one of the prior art described above. Furthermore, with respect to the said the amount of heat released, the value of 2-isopropoxyiminopropanedinitrile is far smaller than the one of 2-amino-2-hydroxyimino-N-hydroxyacetimidoylcyanide of the formula (6), which is the production intermediate in the prior art. This value means the drastically increased safety of the process of the present invention.

Therefore, it was found that a 2-alkoxyiminopropanedinitrile of the general formula (2), which is the novel production intermediate in the present invention, is dramatically safer than 2-amino-2-hydroxyimino-N-hydroxyacetimidoylcyanide of the formula (6), which is the production intermediate in the prior art.

In addition, in the process of the present invention, it is presumed that, after reacting malononitrile of the formula (1) with an alkali metal salt of nitrous acid in the presence of a protic acid, 2-hydroxyiminopropanedinitrile of the following formula (8) and/or a salt of the same was generated, as a precursor to 2-alkoxyiminopropanedinitrile of the general formula (2).

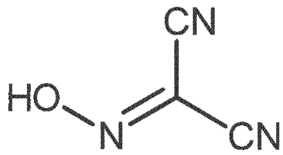

(8)

Specific examples of a salt of 2-hydroxyiminopropanedinitrile of the formula (8) include sodium salt of 2-hydroxyiminopropanedinitrile. It can be said that these compounds are also intermediates for the production in the process of the present invention. Also in the differential scanning calorimetric measurement of these compounds, improved thermal properties are observed as described below. For 2-hydroxyiminopropanedinitrile of the formula (8), an exothermic onset temperature of 147°C and the amount of heat released of 1.6 kJ/g were found. This exothermic onset temperature of 2-hydroxyiminopropanedinitrile is relatively higher, i.e., safer than the one of 2-amino-2-hydroxyimino-N-hydroxyacetimidoylcyanide of the formula (6), which is the production intermediate in the prior art. Furthermore, with respect to the said the amount of heat released, the value of 2-hydroxyiminopropanedinitrile is far smaller than the one in the prior art described above, and is enough to maintain safety. Furthermore, the said sodium salt of 2-hydroxyiminopropanedinitrile exhibits endothermic onset temperature of 222°C and the amount of heat absorbed of -0.9 kJ/g on differential scanning calorimetry. This onset temperature means better thermal stability than the one of the prior art described above. Additionally, the thermal event is exothermic rather than endothermic, i.e., is extremely safer than the one of the prior art described above.

As described above, physical properties of the production intermediates in the present invention show the significantly improved safety in the process of the present invention, i.e., the advantage of the process of the present invention compared to prior art.

In other words, they substantially reduce the risks of dangerous decomposition and the like during industrial manufacture. Therefore, the process of the present invention is safely applicable to production on a larger scale, such as in a pilot plant or in an industrial manufacture.

According to the present invention, a 2-alkoxyiminopropanedinitrile of the general formula (2) can be produced, without limiting an alkylating agent to the one having high reactivity (that is, regardless of species of alkylating agents), without using an expensive silver salt, with a high yield, when malononitrile is reacted with an alkali metal salt of nitrous acid and then the resulting compound is reacted with an alkylating agent. Furthermore, according to the present invention, a 2-alkoxyiminopropanedinitrile of the general formula (2) can be produced, using recyclable and inexpensive solvent (for example, an organic solvent such as ethers or particularly aromatic hydrocarbon derivatives). Therefore, the present invention also provides a novel industrially satisfactory production process for a 2-alkoxyiminopropanedinitrile of the

general formula (2).

From the viewpoints of industrial utility value and the like, when this industrially satisfactory manufacture process for a 2-alkoxyiminopropanedinitrile of the general formula (2) was found, the above-mentioned production route for a 2-amino-2-hydroxyimino-N-alkoxyacetimidoylcyanide of the general formula (3), which route do not use a dangerous intermediate for the production thereof, has only become realized for the first time.

Thus, according to the present invention, the target 2-amino-2-hydroxyimino-N-alkoxyacetimidoylcyanide of the general formula (3), as well as a 2-alkoxyiminopropanedinitrile of the general formula (2) which have preferable thermal properties and is an intermediate for the production thereof, can be produced dramatically safely and using a reagent and solvent which are easily available at a low cost.

Furthermore, the process of the present invention has a high yield, and can be efficiently implemented on an industrial scale, by a simple operation, under mild conditions, and without using a special reaction apparatus. Additionally, in the process of the present invention, it is considered that amounts of waste are small and properties of the waste are preferable.

Therefore, the present invention is economical and also environmentally friendly, and has high industrial use value.

Description of Embodiment

The present invention will be described in detail below.

The present invention solves the above problems by providing inventions according to the following items [1] to [47].

[1] A compound of the general formula (2):

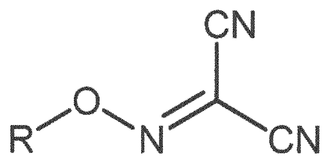

(2)

wherein R is C3 to C6 alkyl group.

[2] The compound according to [1], wherein R is isopropyl or propyl.

[3] The compound according to [1], wherein R is isopropyl.

[4] The compound according to [1], wherein R is propyl.

[5] A process for the production of a compound of the general formula (3) :

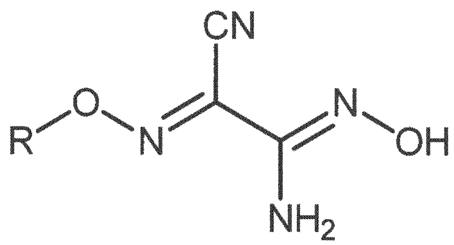

(3)

wherein R is C3 to C6 alkyl group,
which comprises reacting a compound of the general formula (2) :

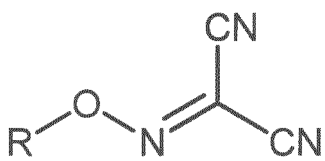

(2)

wherein R is as defined above,
with a hydroxylamine compound.

[6] A process for the production of a compound of the general formula (3) :

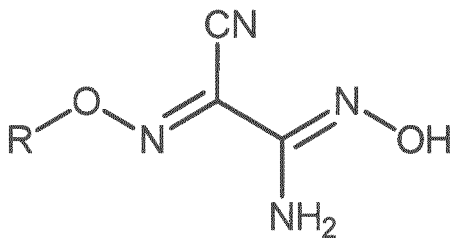

(3)

wherein R is C3 to C6 alkyl group, which comprises the following steps;

(i) reacting malononitrile of the formula (1):

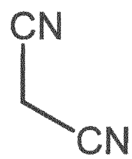

(1)

with an alkali metal salt of nitrous acid in the presence of a protic acid;

(ii) reacting the product of step (i) with C3 to C6 alkyl halide in the presence of a phase transfer catalyst to produce a compound of the general formula (2):

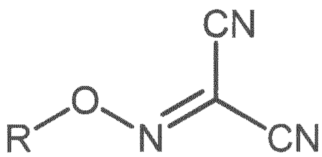

(2)

wherein R is as defined above; and

(iii) reacting the resulting compound of the general formula (2) with a hydroxylamine compound.

[7] The process according to [6], wherein the reaction of the step (ii) is carried out in a solvent system consisting of aromatic hydrocarbon derivatives and water or in a solvent system consisting comprised of ethers and water.

[8] The process according to [6], wherein the reaction of the step (ii) is carried out in a solvent system consisting of aromatic hydrocarbon derivatives and water.

[9] The process according to [6], wherein the reaction of the step (ii) is carried out in a solvent system consisting of ethers and water.

[10] The process according to [6], wherein the reaction of the step (ii) is carried out in a solvent system consisting of one or more aromatic hydrocarbon derivatives selected from the group consisting of toluene, xylenes and chlorobenzene, and water.

[11] The process according to [6], wherein the reaction of the step (ii) is carried out in a solvent system consisting of toluene and water.

[12] The process according to [6], wherein the reaction of the step (ii) is carried out in a solvent system consisting of tetrahydrofuran and water.

[13] The process according to any one of [6] to [12], wherein R is isopropyl or propyl.

[14] The process according to any one of [6] to [12], wherein R is isopropyl.

[15] The process according to any one of [6] to [12], wherein R is propyl.

[16] A process for the production of a compound of the general formula (2):

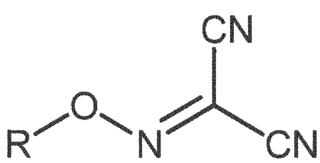

(2)

wherein R is C3 to C6 alkyl group,

which comprises the following steps ;

(i) reacting malononitrile of the formula (1):

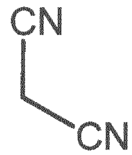

( 1 )

with an alkali metal salt of nitrous acid in the presence of a protic acid; and
(ii) reacting the product of step (i) with C3 to C6 alkyl halide in the presence of a phase transfer catalyst.

[17] The process according to [16], wherein the reaction of the step (ii) is carried out in a solvent system consisting of aromatic hydrocarbon derivatives and water or in a solvent system consisting of ethers and water.
[18] The process according to [16], wherein the reaction of the step (ii) is carried out in a solvent system consisting of aromatic hydrocarbon derivatives and water.
[19] The process according to [16], wherein the reaction of the step (ii) is carried out in a solvent system consisting of ethers and water.
[20] The process according to [16], wherein the reaction of the step (ii) is carried out in a solvent system consisting of one or more aromatic hydrocarbon derivatives selected from the group consisting of toluene, xylenes and chlorobenzene, and water.
[21] The process according to [16], wherein the reaction of the step (ii) is carried out in a solvent system consisting of toluene and water.
[22] The process according to [16], wherein the reaction of the step (ii) is carried out in a solvent system consisting of tetrahydrofuran and water.
[23] The process according to any one of [16] to [22], wherein R is isopropyl or propyl.
[24] The process according to any one of [16] to [22], wherein R is isopropyl.
[25] The process according to any one of [16] to [22], wherein R is propyl.
[26] A process for the production of a compound of the general formula (3):

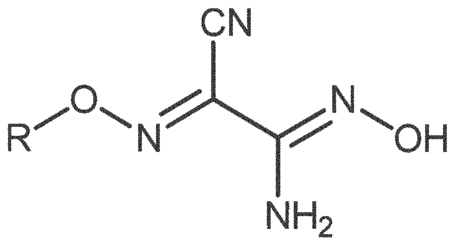

( 3 )

wherein R is C3 to C6 alkyl group,
which comprises the following steps;

(i) reacting malononitrile of the formula (1):

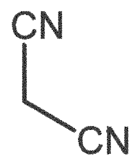

( 1 )

with an alkali metal salt of nitrous acid in the presence of a protic acid to produce a compound of the general formula (4):

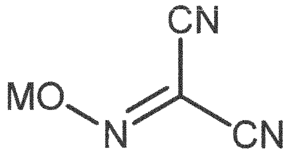

(4)

wherein M is hydrogen or alkali metal, or mixture thereof; and

(ii) reacting the compound of the general formula (4) with C3 to C6 alkyl halide in the presence of a phase transfer catalyst to produce a compound of the general formula (2):

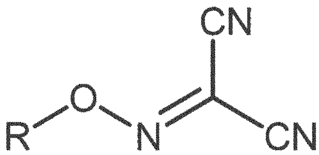

(2)

wherein R is as defined above;

(iii) reacting the resulting compound of the general formula (2) obtained in the step (ii) with a hydroxylamine compound.

[27] The process according to [26], wherein the reaction of the step (ii) is carried out in a solvent system consisting of aromatic hydrocarbon derivatives and water or in a solvent system consisting of ethers and water.

[28] The process according to [26], wherein the reaction of the step (ii) is carried out in a solvent system consisting of aromatic hydrocarbon derivatives and water.

[29] The process according to [26], wherein the reaction of the step (ii) is carried out in a solvent system consisting of ethers and water.

[30] The process according to [26], wherein the reaction of the step (ii) is carried out in a solvent system consisting of one or more aromatic hydrocarbon derivatives selected from the group consisting of toluene, xylenes and chlorobenzene, and water.

[31] The process according to [26], wherein the reaction of the step (ii) is carried out in a solvent system consisting of toluene and water.

[32] The process according to [26], wherein the reaction of the step (ii) is carried out in a solvent system consisting of tetrahydrofuran and water.

[33] The process according to any one of [26] to [32], wherein R is isopropyl or propyl.

[34] The process according to any one of [26] to [32], wherein R is isopropyl.

[35] The process according to any one of [26] to [32], wherein R is propyl.

[36] A process for the production of a compound of the general formula (2):

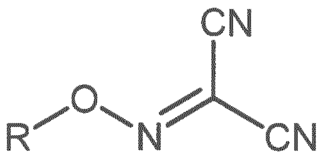

(2)

wherein R is C3 to C6 alkyl group,
which comprises the following steps;

(i) reacting malononitrile of the formula (1):

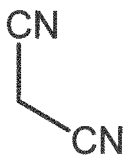

(1)

with an alkali metal salt of nitrous acid in the presence of a protic acid to produce a compound of the general formula (4):

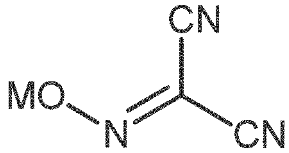

(4)

wherein M is hydrogen or alkali metal, or mixture thereof; and
(ii) reacting the compound of the general formula (4) with C3 to C6 alkyl halide in the presence of a phase transfer catalyst.

[37] The process according to [36], wherein the reaction of the step (ii) is carried out in a solvent system consisting of aromatic hydrocarbon derivatives and water or in a solvent system consisting of ethers and water.
[38] The process according to [36], wherein the reaction of the step (ii) is carried out in a solvent system consisting of aromatic hydrocarbon derivatives and water.
[39] The process according to [36], wherein the reaction of the step (ii) is carried out in a solvent system consisting of ethers and water.
[40] The process according to [36], wherein the reaction of the step (ii) is carried out in a solvent system consisting of one or more aromatic hydrocarbon derivatives selected from the group consisting of toluene, xylenes and chlorobenzene, and water.
[41] The process according to [36], wherein the reaction of the step (ii) is carried out in a solvent system consisting of toluene and water.
[42] The process according to [36], wherein the reaction of the step (ii) is carried out in a solvent system consisting of tetrahydrofuran and water.
[43] The process according to any one of [36] to [42], wherein R is isopropyl or propyl.
[44] The process according to any one of [36] to [42], wherein R is isopropyl.
[45] The process according to any one of [36] to [42], wherein R is propyl.
[46] The process according to any one of [6] to [45], wherein pH of a solvent system in the step (ii) is in the range of 6 to 7.
[47] The process according to any one of [6] to [45], wherein pH of a solvent system in the step (ii) is controlled into the range of 6 to 7 by addition of a base.

The terms and symbols used in the present specification will be explained below.

"Ca to Cb" means that the number of carbon is a to b. For example, "C3 to C6" in "C3 to C6 alkyl" means that the number of carbon atoms in the alkyl is 3 to 6.

Examples of the alkyl group include C3 to C6 alkyl group, preferably C3 alkyl group. The C3 to C6 alkyl group means a strait or branched chain alkyl group having 3 to 6 carbon atoms. The C3 to C6 alkyl group includes, specifically, for example, propyl, isopropyl, butyl, sec-butyl, isobutyl, tert-butyl, pentyl, hexyl and the like, preferably propyl, isopropyl, butyl, sec-butyl, isobutyl and tert-butyl, more preferably propyl and isopropyl. However, it is not limited to these examples. The C3 alkyl group means a strait or branched chain alkyl group having 3 carbon atoms. The C3 alkyl group includes, specifically, for example, propyl and isopropyl.

Examples of the alkoxy group include C3 to C6 alkoxy group, preferably C3 alkoxy group. The C3 to C6 alkoxy group means a (C3 to C6 alkyl)-O- group wherein the C3 to C6 alkyl has the same meaning as the above-mentioned C3 to C6 alkyl group. The C3 to C6 alkoxy group includes, specifically, for example, propoxy, isopropoxy, butoxy, sec-butoxy, isobutoxy, tert-butoxy, pentyloxy, hexyloxy and the like, preferably propoxy, isopropoxy, butoxy, sec-butoxy, isobutoxy, tert-butoxy, more preferably propoxy, isopropoxy. However, it is not limited to these examples. The C3 alkoxy group means a (C3 alkyl)-O- group wherein the C3 alkyl has the same meaning as the above-mentioned C3 alkyl group. The C3 alkoxy group includes, specifically, for example, propoxy and isopropoxy.

Examples of the aromatic hydrocarbon derivatives include benzene, toluene, xylenes, ethyl benzene, cumene, trimethyl benzene, methyl isopropyl benzene, methyl naphthalene, dimethyl naphthalene, ethyl naphthalene chlorobenzene, dichlorobenzene, trichlorobenzene, tetrachlorobenzene nitrobenzene and the like, preferably toluene, xylenes, chlorobenzene, dichlorobenzene, nitrobenzene, more preferably toluene, xylenes, chlorobenzene, and further preferably toluene.

Examples of the aliphatic hydrocarbon compounds include hexane, heptane, octane, isooctane, nonane, decane, undecane, dodecane, isododecane, tridecane, tetradecane, pentadecane, hexadecane, isohexadecane, heptadecane, octadecane, nonadecane, eicosane, isoeicosane, triacontane, cyclohexane, ethylcyclohexane, methyl isopropyl cyclohexane , methyl decalin, dimethyl decalin, ethyl decalin and the like.

Examples of the halogenated aliphatic hydrocarbon compounds include dichloromethane, chloroform, carbon tetrachloride, 1,2-dichloroethane, 1,3-dichloropropane and the like.

Examples of the ethers include tetrahydrofuran (THF), 2-methyltetrahydrofuran, diethyl ether, di-n-propyl ether,

diisopropyl ether, dibutyl ether, di-tert-butyl ether, diphenyl ether, cyclopentyl methyl ether (CPME), methyl-tert-butyl ether, 1,2-dimethoxyethane (DME), diglyme, triglyme, 1,4-dioxane and the like, preferably tetrahydrofuran (THF), diisopropyl ether, dibutyl ether, cyclopentyl methyl ether (CPME), methyl-tert-butyl ether and more preferably tetrahydrofuran.

Examples of the alcohols include methanol, ethanol, propanol, 2-propanol, butanol, tert-butyl alcohol, pentanol, hexanol, heptanol, octanol, cyclohexanol, ethylene glycol, propylene glycol and the like.

Examples of the nitriles include acetonitrile, propionitrile, benzonitrile and the like.

Examples of the amides include N,N-dimethylformamide (DMF), N,N-diethyl formamide, N,N-dimethylacetamide (DMAC), N,N-diethylacetamide, N-methylpyrrolidone (NMP) and the like.

Examples of the alkyl ureas include tetramethyl urea, N,N'-dimethyl imidazolidinone (DMI) and the like.

Examples of the sulfoxides include dimethyl sulfoxide (DMSO) and the like.

Examples of the sulfones include sulfolane, dimethyl sulfone and the like.

Examples of the carbonic acid esters include ethylene carbonate, propylene carbonate and the like.

Examples of the ketones include acetone, ethyl methyl ketone, isopropyl methyl ketone, isobutyl methyl ketone (MIBK), cyclohexanone and the like.

Examples of the carboxylate esters include methyl acetate, ethyl acetate, propyl acetate, butyl acetate and the like.

Examples of the carboxylic acids include formic acid, acetic acid, propionic acid and the like.

Examples of the nitrate aromatic hydrocarbons include nitrobenzene and the like.

Examples of aromatic heterocycles include pyridine and the like.

Examples of the alkali metal hydroxides include lithium hydroxide, sodium hydroxide, potassium hydroxide and the like.

Examples of the alkali earth metal hydroxides include magnesium hydroxide, calcium hydroxide, barium hydroxide and the like.

Examples of the alkali metal carbonates include lithium carbonate, sodium carbonate, potassium carbonate and the like.

Examples of the alkali earth metal carbonates include magnesium carbonate, calcium carbonate, barium carbonate and the like.

Examples of the alkali metal hydrogen carbonates include lithium hydrogen carbonate, sodium hydrogen carbonate, potassium hydrogen carbonate and the like.

Examples of the alkali earth metal hydrogen carbonates include magnesium hydrogen carbonate, calcium hydrogen carbonate, barium hydrogen carbonate and the like.

Examples of the phosphates include sodium phosphate, potassium phosphate, calcium phosphate and the like.

Examples of the hydrogen phosphates include disodium hydrogen phosphate, dipotassium hydrogen phosphate, calcium hydrogen phosphate and the like.

Examples of the pyridines include pyridine, 4--dimethylaminopyridine, 4-pyrrolidinopyridine, 2,6-lutidine and the like.

Examples of the quinolines include quinoline and the like.

Examples of the isoquinolines include isoquinoline and the like.

Examples of the tertiary amines include trimethyl amine, triethyl amine, tributyl amine, diisopropylethyl amine, triisopropyl amine and the like.

Examples of the secondary amines include diethyl amine, dipropyl amine, diisopropyl amine and the like.

Examples of the primary amines include propyl amine, butyl amine and the like.

Examples of the aromatic amines include aniline, N,N-dimethyl aniline, N,N-diethyl aniline and the like.

Examples of the cyclic amines include pyrrolidine, piperidine, morpholine, N-methylmorpholine, piperazine, 1,8-diazabicyclo[5.4.0]-7-undec-7-ene (DBU), 1,5-diazabicyclo[4.3.0]non-5-ene (DBN), 1,4-diazabicyclo[2.2.2]octane (DABCO) and the like.

Examples of the carboxylic acid alkali metal salts include sodium formate, potassium formate, lithium acetate, sodium acetate, potassium acetate, sodium propionate, potassium propionate and the like.

Examples of the carboxylic acid alkali earth metal salts include magnesium acetate, magnesium propionate, calcium acetate, calcium propionate and the like.

(Step (i))

Firstly, the step (i) will be described.

The step (i) is the reaction of malononitrile presented by the formula (1) with an alkali metal salt of nitrous acid in presence of a protic acid.

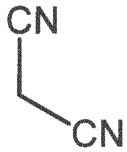

(1)

(Raw material; malononitrile)

As the raw material of the method of the present invention, malononitrile presented by the formula (1) is used. Malononitrile is a known compound and is industrially available at relatively low price. Furthermore, it is a preferable industrial raw material in terms of handling.

(Alkali metal salts of nitrous acid)

Examples of alkali metal salts of nitrous acid include sodium nitrite, potassium nitrite, lithium nitrite and the like. However, they are not limited to these examples. From the viewpoints of price, availability and the like, sodium nitrite or potassium nitrite are preferable. Sodium nitrite is more preferable.

As for the form of alkali metal salts of nitrous acid, the salts can be used in any form as long as the reaction proceeds. For example, the form includes a solid of an alkali metal salt of nitrous acid only, an aqueous solution or a solution of any other solvents except water at any concentration. Additionally, an alkali metal salt of nitrous acid can be used alone or as a mixture of two or more these salts at any mixing ratios can also be used.

(Amount of alkali metal salts of nitrous acid to be used)

Alkali metal salts of nitrous acid can be used in any amount as long as the reaction proceeds. From the viewpoints of yield, inhibition of by-product, economic efficiency and the like, they can be usually used, for example, in a range of 0.8 to 2.0 mol, preferably in a range of 0.9 to 1.5 mol and more preferably in a range of 0.9 to 1.2 mol to 1 mol of malononitrile presented by the formula (1).

(Protic acids)

Examples of protic acids include halogenated hydracids, sulfuric acid, nitric acid, phosphoric acid, poly phosphoric acid, carboxylic acid, sulfonic acid and the like. However, they are not limited to these examples.

Examples of halogenated hydracids include hydrochloric acid, hydrobromic acid, hydrofluoric acid and the like.

Examples of carboxylic acids include formic acid, acetic acid, propionic acid, trifluoroacetic acid, benzoic acid and the like.

Examples of sulfonic acids include methane sulfonic acid, ethane sulfonic acid, trifluoromethane sulfonic acid, benzene sulfonic acid, p-toluene sulfonic acid and the like.

From the viewpoints of price, availability, reactivity and the like, halogenated hydracids or carboxylic acids are preferable. Hydrochloric acid or acetic acid are more preferable.

As for the form of protic acids, the acids can be used in any form as long as the reaction proceeds. For example, the form includes a solid or liquid of protic acids only, an aqueous solution or a solution from any other solvents except water at any concentration and the like. Additionally, a protic acid can be used alone or as a mixture of two or more these acids at any mixing ratios.

(Amount of protic acids to be used)

Protic acids can be used in any amount as long as the reaction proceeds. From the viewpoints of yield, inhibition of by-product, economic efficiency and the like, they can be usually used, for example, in a range of 0.005 to 3.0 mol, preferably a range of 0.01 to 2.0 mol, more preferably in a range of 0.05 to 1.5 mol and further preferably in a range of 0.05 to 1.3 mol to 1 mol of malononitrile presented by the formula (1). In addition, the especially preferable range in which protic acids are used depends on the acids. For example, it is 0.9 to 1.2 mol when the protic acid is hydrochloric acid and 0.05 to 1.2 mol when the protic acid is acetic acid.

(The solvents for the step (i))

The reaction of the step (i) can be conducted without any solvent. However, solvents may be used from the viewpoints of its smooth proceeding and the like. Any solvent can be used as long as the reaction of the step (i) proceeds

and there is no negative effect on the proceeding of the reactions of the steps (ii) and (iii) . Examples of solvents which can be used in the step (i) include water, ethers, alcohols, nitriles, amides, alkyl ureas, sulfoxides, sulfones, carbonate esters, ketones, carboxylic acid esters, carboxylic acids, nitrated aliphatic hydrocarbons, aromatic heterocycles, mixtures of these solvents at any mixing ratio and the like. However, they are not limited to these examples.

From the viewpoints of price, easy handling and the like, the solvents for use in the step (i) are preferably for example, ethers, alcohols, nitriles, amides, alkyl ureas, sulfoxides, sulfones, water and mixtures of these solvents and preferably ethers, alcohols, nitriles, amides, water and mixtures of these solvents.

The specific examples of preferable solvents for use in the step (i) include tetrahydrofuran (THF), dipropyl ether, diisopropyl ether, dibutyl ether, cyclopentyl methyl ether (CPME), methyl-tert-butyl ether, methanol, ethanol, propanol, 2-propanol, butanol, acetonitrile, N,N-dimethyl formamide (DMF), N,N-dimethyl acetamide (DMAC), N-methyl pyrrolidone (NMP), N,N'-dimethyl imidazolidinone (DMI) dimethyl sulfoxide (DMSO), sulfolane, water and mixtures of these solvents. The specific examples of more preferable solvents include tetrahydrofuran (THF), diisopropyl ether, dibutyl ether, cyclopentyl methyl ether (CPME), methyl-tert-butyl ether, methanol, ethanol, propanol, 2-propanol, butanol, acetonitrile, N,N-dimethyl formamide (DMF), N,N-dimethyl acetamide (DMAC), N-methyl pyrrolidone (NMP), water and mixtures of these solvents. Water can be exemplified as the further preferable solvent.

(The amount of solvent to be used in the step (i))

The solvent can be used in any amount in the step (i) as long as the reaction system can be stirred thoroughly. From the viewpoints of reactivity, inhibition of by-product and economic efficiency and the like, it can be usually used, for example, in a range of 0 to 10.0 L, preferably in a range of 0.01 to 5.0 L, more preferably in a range of 0.05 to 3.0 L and further preferably in a range of 0.1 to 2.0 L to 1 mol of malononitrile presented by the formula (1).

(Reaction temperature of the step (i))

There is no limitation in reaction temperature of the step (i). From the viewpoints of yield, inhibition of by-product, economic efficiency and the like, usually the range of -30° C (minus 30° C) to 90° C, preferably the range of -20° C (minus 20° C) to 70° C, more preferably the range of -10° C (minus 10° C) to 30°C and further preferably the range of -5° C (minus 5° C) to 20°C can be exemplified.

(Reaction time of the step (i))

There is no limitation in reaction time of the step (i). From the viewpoints of yield, inhibition of by-product, economic efficiency and the like, usually the range of 0.5 to 48 hours, preferably 0.5 to 24 hours and more preferably the range of 0.5 to 12 hours can be exemplified.

(Product of the step (i))

The form of "the product of the step (i)" in the present invention includes the reaction mixture of the step (i) and when possible, the crude product, which is isolated but not purified reaction product of the step (i) and the substance, which is isolated and purified reaction product of the step (i) and the like. But it is not limited to them. Therefore, there is no limitation in the form of the product of the step (i) for use in the step (ii). However, from the viewpoints of economic efficiency, safety and the like, the reaction mixture of the step (i) is preferably used for the step (ii).

The mechanism and the like of the reaction in the present invention are not clear. However, the product of the step (i) is presumed to be 2-hydroxyiminopropanedinitrile presented by the formula (8) below or its salt or mixture thereof when considering the present invention after its completion.

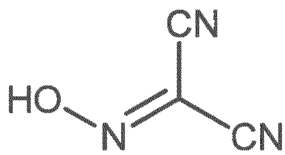

(8)

In other words, the product of the step (i) is presumed to be the compound presented by the general formula (4),

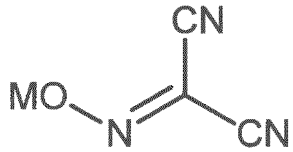

(4)

wherein M is hydrogen or alkali metal, or mixture thereof.

Examples of the alkali metals include sodium, potassium, lithium and the like.

The description on the form of the above compound presented by the above general formula (4) is equivalent to that on "the product of the step (i)".

(Step (ii))

Secondly, the step (ii) will be described.

The step (ii) is the reaction of the product of the step (i) with a C3 to C6 alkyl halide in presence of a phase transfer catalyst to produce a compound presented:

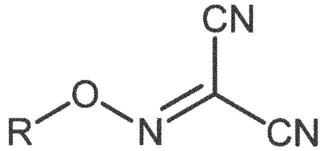

(2)

wherein R is C3 to C6 alkyl group.

(Phase transfer catalysts)

Examples of the phase transfer catalysts include quaternary ammonium salts, quaternary phosphonium salts, crown ethers and the like. However, they are not limited to these examples.

Examples of the quaternary ammonium salts include tetramethyl ammonium chloride, tetramethyl ammonium bromide, tetraethyl ammonium chloride, tetraethyl ammonium bromide, tetrabutyl ammonium fluoride, tetrabutyl ammonium chloride, tetrabutyl ammonium bromide, tetrabutyl ammonium iodide, tetrabutyl ammonium hydrogen sulfate, tetrabutyl ammonium hydroxide, benzyl trimethyl ammonium chloride, benzyl trimethyl ammonium bromide, benzyl trimethyl ammonium hydroxide, benzyl triethyl ammonium chloride, benzyl triethyl ammonium bromide, octyl trimethyl ammonium chloride, octyl trimethyl ammonium bromide, trioctyl methyl ammonium chloride, trioctyl methyl ammonium bromide, trioctyl ethyl ammonium chloride, trioctyl ethyl ammonium bromide, lauryl trimethyl ammonium chloride (dodecyl trimethyl ammonium chloride), lauryl trimethyl ammonium bromide (dodecyl trimethyl ammonium bromide), benzyl lauryl dimethyl ammonium chloride (benzyl dodecyl dimethyl ammonium chloride), benzyl lauryl dimethyl ammonium bromide (benzyl dodecyl dimethyl ammonium bromide), myristyl trimethyl ammonium chloride (tetradecyl trimethyl ammonium chloride), myristyl trimethyl ammonium bromide (tetradecyl trimethyl ammonium bromide), benzyl dimethyl stearyl ammonium chloride (benzyl octadecyl dimethyl ammonium chloride), benzyl dimethyl stearyl ammonium bromide (benzyl octadecyl dimethyl ammonium bromide) and the like.

Examples of the crown ethers include 12-crown-4, 15-crown-5, 18-crown-6 and the like.

Examples of the quaternary phosphonium salts include tetrabutyl phosphonium bromide, tetraoctyl phosphonium bromide, tetraphenyl phosphonium bromide and the like.

From the viewpoints of price, availability, reactivity and the like, phase transfer catalysts are preferably quaternary ammonium salts, more preferably tetrabutyl ammonium bromide, tetrabutyl ammonium hydrogen sulfate, benzyl trimethyl ammonium chloride, benzyl trimethyl ammonium bromide, trioctyl methyl ammonium chloride, trioctyl methyl ammonium bromide, benzyl lauryl dimethyl ammonium chloride, myristyl trimethyl ammonium bromide, benzyl dimethyl stearyl ammonium chloride, benzyl dimethyl stearyl ammonium bromide and the like, further preferably tetrabutyl ammonium bromide, benzyl trimethyl ammonium chloride and especial preferably tetrabutyl ammonium bromide.

As for the form of phase transfer catalyst, the catalyst can be used in any form as long as the reaction proceeds. For example, the form includes a solid or a liquid of the phase transfer catalyst only, an aqueous solution or a solution of any other solvents except water at any concentration and the like. Additionally, it can also be used alone or as a mixture of two or more catalysts at any mixing ratios.

(The amount of phase transfer catalyst to be used)

The phase transfer catalyst can be used in any amount as long as the reaction proceeds. From the viewpoints of yield, inhibition of by-product, economic efficiency and the like, they can be usually used, for example, in a range of 0.001 to 1.0 mol, preferably in a range of 0.005 to 0.3 mol and more preferably in a range of 0.01 to 0.2 mol to 1 mol of malononitrile presented by the formula (1).

(C3 to C6 alkyl halides)

The C3 to C6 alkyl halides mean strait or branched chain alkyl halides having 3 to 6 carbon atoms. They include, for example, propyl chloride, isopropyl chloride, butyl chloride, sec-butyl chloride, isobutyl chloride, tert-butyl chloride, pentyl chloride, 1-methylbutyl chloride, 2-methylbutyl chloride, 3-methylbutyl chloride, 1-ethylpropyl chloride, 1,1-dimethylpropyl chloride, 1,2-dimethylpropyl chloride, 2,2-dimethylpropyl chloride, hexyl chloride, 1-methylpentyl chloride, 2-methylpentyl chloride, 3-methylpentyl chloride, 4-methylpentyl chloride, 1,2-dimethylbutyl chloride, 1,3-dimethylbutyl chloride, 1-ethylbutyl chloride, 2-ethylbutyl chloride, 1-methyl-2,2-dimethylpropyl chloride, 1,1-dimethyl-2-methylpropyl chloride, 1-ethyl-1-methylpropyl chloride, propyl bromide, isopropyl bromide, butyl bromide, sec-butyl bromide, isobutyl bromide, tert-butyl bromide, pentyl bromide, 1-methylbutyl bromide, 2-methylbutyl bromide, 3-methylbutyl bromide, 4-methylpentyl bromide, 1-ethylpropyl bromide, 1,1-dimethylpropyl bromide, 1,2-dimethylpropyl bromide, 2,2-dimethylpropyl bromide, hexyl bromide, 1-methylpentyl bromide, 2-methylpentyl bromide, 3-methylpentyl bromide, 1,2-dimethylbutyl bromide, 1,3-dimethylbutyl bromide, 1-ethylbutyl bromide, 2-ethylbutyl bromide, 1-methyl-2,2-dimethylpropyl bromide, 1,1-dimethyl-2-methylpropyl bromide, 1-ethyl-1-methylpropyl bromide, propyl iodide, isopropyl iodide, butyl iodide, sec-butyl iodide, isobutyl iodide, tert-butyl iodide, pentyl iodide, 1-methylbutyl iodide, 2-methylbutyl iodide, 3-methylbutyl iodide, 1-ethylpropyl iodide, 1,1-dimethylpropyl iodide, 1,2-dimethylpropyl iodide, 2,2-dimethylpropyl iodide, hexyl iodide, 1-methylpentyl iodide, 2-methylpentyl iodide, 3-methylpentyl iodide, 4-methylpentyl iodide, 1,2-dimethylbutyl iodide, 1,3-dimethylbutyl iodide, 1-ethylbutyl iodide, 2-ethylbutyl iodide, 1-methyl-2,2-dimethylpropyl iodide, 1,1-dimethyl-2-methylpropyl iodide, 1-ethyl-1-methylpropyl iodide and the like.

From the viewpoints of price, availability, reactivity, utility of the product and the like, C3 to C6 alkyl halides are preferably propyl chloride, isopropyl chloride, butyl chloride, sec-butyl chloride, isobutyl chloride, tert-butyl chloride, propyl bromide, isopropyl bromide, butyl bromide, sec-butyl bromide, isobutyl bromide, tert-butyl bromide, more preferably propyl chloride, isopropyl chloride, propyl bromide, isopropyl bromide and further preferably propyl bromide and isopropyl bromide.

(The amount of C3 to C6 alkyl halides to be used)

C3 to C6 alkyl halides can be used in any amount as long as the reaction proceeds. From the viewpoints of yield, inhibition of by-product, economic efficiency and the like, they can be usually used, for example, in a range of 0.8 to 3.0 mol, preferably in a range of 0.9 to 2.0 mol and more preferably in a range of 0.9 to 1.5 mol to 1 mol of malononitrile presented by the formula (1).

(pH of the step (ii))

For the smooth proceeding of the reaction of the step (ii), pH of the solvent system may be adjusted. The pH may or may not be adjusted as long as the reaction proceeds smoothly. Furthermore, the adjustment of pH can be carried out at any point of the step (ii) as long as the reaction proceeds smoothly. For example, the pH may be adjusted before or during the addition of C3 to C6 alkyl halide.

As for the pH of the solvent system of the step (ii), any pH range is possible as long as the reaction proceeds. From the viewpoints of yield, inhibition of by-product, economic efficiency and the like, usually the pH range of 4 to 9, preferably 4 to 8, more preferably 5 to 8, further preferably 5 to 7 and especially preferably 6 to 7 can be exemplified.

(The base of the step (ii))

A base can be used for adjustment of pH of solvent system in the step (ii) as described above. The mechanism and the like of the reactions of the present invention are not clear. However, as a result of the consideration after the completion of the present invention, it is presumed as follows. It is presumed to be necessary to use a base when the protic acid used in the step (i) is as stronger as acetic acid or stronger than acetic acid. And it is presumed to be especially necessary to use a base when the protic acid used in the step (i) is as stronger as acetic acid or stronger than acetic acid and used in the amount of more than 0.1 equivalents to 1 mol of malononitrile.

Bases which can be used in the step (ii) are for example, inorganic bases such as alkali metal hydroxides,

alkali earth metal hydroxides, alkali metal carbonates, alkali earth metal carbonates, alkali metal hydrogen carbonates, alkali earth metal hydrogen carbonates and the like, organic bases such as pyridines, quinolines, isoquinolines, tertiary amines, secondary amines, primary amines, aromatic amines, cyclic amines, alkali metal carboxylates, alkali earth metal carboxylates and the like. However, they are not limited to these examples.

From the viewpoints of reactivity, yield, price, easy handling and the like, the examples of bases used in the step (ii) are preferably alkali metal hydroxides, alkali metal carbonates, alkali metal hydrogen carbonates, tertiary amines, alkali metal carboxylates, more preferably alkali metal hydroxides, alkali metal carbonates, alkali metal hydrogen carbonates and further preferably alkali metal hydroxides. The specific examples of bases used in the step (ii) are preferably sodium hydroxide, potassium hydroxide, sodium carbonate, potassium carbonate, sodium hydrogen carbonate, potassium hydrogen carbonate, triethyl amine, sodium acetate, potassium acetate, more preferably sodium hydroxide, potassium hydroxide, sodium carbonate, potassium carbonate, sodium hydrogen carbonate, potassium hydrogen carbonate and further preferably sodium hydroxide and potassium hydroxide.

As for the form of the bases for use in the step (ii), the bases can be used in any form as long as the reaction proceeds. For example, the form includes a solid or a liquid of a base only, an aqueous solution or a solution from any other solvents except water at any concentration and the like. Additionally, a base can be used alone or as a mixture of two or more bases at any mixing ratios.

(The amount of base used in the step (ii))

A base may or may not be used in the step (ii) as long as the reaction proceeds. In case of using a base in the step (ii), it is used in such amount so that the pH of solvent system can be adjusted to the ranges of usually 4 to 9, preferably 4 to 8, more preferably 5 to 8, further preferably 5 to 7 and especially preferably 6 to 7 from the viewpoints of yield, inhibition of by-product, economic efficiency and the like.

(The solvent system in the step (ii))

The solvent of the step (ii) will be described as follows. The solvent system for use in the step (ii) includes for example, aromatic hydrocarbon derivatives, aliphatic hydrocarbons, halogenated aliphatic hydrocarbons, ethers, alcohols, nitriles, amides, alkyl ureas, sulfoxides, sulfones, ketones, carboxylic acid esters, carboxylic acids, aromatic heterocycles, water and mixtures of these solvents at any mixing ratios.

From the viewpoints of price, easy handling, reactivity, yield and the like, the preferable solvent system for use in the step (ii) includes for example, aromatic hydrocarbon derivatives, halogenated aliphatic hydrocarbons, ethers, alcohols, nitriles, amides, alkyl ureas, sulfoxides, sulfones, water and mixtures consisting of these solvents. The more preferable solvent system includes for example, aromatic hydrocarbon derivatives, halogenated aliphatic hydrocarbons, ethers, alcohols, nitriles, amides, water and mixtures consisting of these solvents. The further preferable solvent system includes for example, a solvent system consisting of aromatic hydrocarbon derivatives and water and a solvent system consisting of ethers and water.

The specific examples of preferable solvent system used in the step (ii) are toluene, xylenes, chlorobenzene, dichlorobenzene, nitrobenzene, dichloromethane, tetrahydrofuran (THF), diisopropyl ether, dibutyl ether, cyclopentyl methyl ether (CPME), methyl-tert-butyl ether, methanol, ethanol, propanol, 2-propanol, butanol, acetonitrile , N,N-dimethylformamide (DMF), N,N-dimethylacetamide (DMAC), N-methyl pyrrolidone (NMP), N,N'-dimethyl imidazolidinone (DMI), dimethyl sulfoxide (DMSO), sulfolane, water and solvent systems consisting of these solvents. The specific examples of more preferable solvent system are toluene, xylenes, chlorobenzene, dichlorobenzene, nitrobenzene, dichloromethane, tetrahydrofuran (THF), diisopropyl ether, dibutyl ether, cyclopentyl methyl ether (CPME), methyl-tert-butyl ether, methanol, ethanol, propanol, 2-propanol, butanol, acetonitrile , N,N-dimethylformamide (DMF), N,N-dimethylacetamide (DMAC), N-methyl pyrrolidone (NMP), water and solvent systems consisting of these solvents. The specific examples of further preferable solvent system are solvent systems consisting of water and one or more aromatic hydrocarbon derivatives selected from the group consisting of toluene, xylenes, chlorobenzene, dichlorobenzene, nitrobenzene and solvent systems consisting of water and one or more ethers selected from the group consisting of tetrahydrofuran, diisopropyl ether, dibutyl ether, cyclopentyl methyl ether (CPME), methyl-tert-butyl ether. The specific examples of further preferable solvent system are solvent system consisting of water and one or more aromatic hydrocarbon derivatives selected from the group consisting of toluene, xylenes, chlorobenzene and the solvent system consisting of tetrahydrofuran and water. The specific example of further preferable solvent system is the solvent system consisting of toluene and water.

(The amount of solvent used in the step (ii))

The solvent can be used in any amount to form the solvent system in the step (ii) as long as the reaction system

can be stirred thoroughly. From the viewpoints of reactivity, inhibition of by-product, economic efficiency and the like, water for example, can be usually used in a range of 0 to 10.0 L, preferably in a range of 0.01 to 10.0 L, more preferably in a range of 0.1 to 5.0 L and further preferably in a range of 0.2 to 3.0 L to 1 mol of malononitrile presented by the formula (1). Furthermore, from the same points of view, other solvents except water which are described above for example, can be usually used in a range of 0 to 10.0 L, preferably in a range of 0.01 to 10.0 L, more preferably in a range of 0.1 to 5.0 L and further preferably in a range of 0.2 to 3.0 L to 1 mol of malononitrile presented by the formula (1). It should be noted that mixtures of water and other solvent can be used at any mixing ratio as long as the reaction proceeds. And mixtures consisting of two or more solvents except water can also be used at any mixing ratio as long as the reaction proceeds.

(Reaction temperature of the step (ii))

There is no limitation in reaction temperature of the step (ii) . From the viewpoints of yield, inhibition of by-product, economic efficiency and the like, usually the temperature the range of 10 to 100° C, preferably the range of 40 to 95°C, more preferably the range of 50 to 95° C and further preferably the range of 55 to 90°C can be exemplified.

(Reaction time of the step (ii))

There is no limitation in reaction time of the step (ii). From the viewpoints of yield, inhibition of by-product, economic efficiency and the like, usually the range of 0.5 to 48 hours, preferably the range of 0.5 to 24 hours, more preferably the range of 1-12 hours can be exemplified.

(The product of the step (ii); 2-alkoxyiminopropanedinitrile presented by the general formula (2))

2-alkoxyiminopropanedinitrile of the general formula (2) obtained in the step (ii) is a novelcompound.

The specific examples of 2-alkoxyiminopropanedinitrile presented by the general formula (2) include

2-propoxyiminopropanedinitrile,
2-isopropoxyiminopropanedinitrile,
2-butoxyiminopropanedinitrile,
2-sec-butoxyiminopropanedinitrile,
2-isobutoxyiminopropanedinitrile,
2-tert-butoxyiminopropanedinitrile,
2-pentyloxyiminopropanedinitrile,
2-(1-methylbutyloxyimino)propanedinitrile,
2-(2-methylbutyloxyimino)propanedinitrile,
2-(3-methylbutyloxyimino)propanedinitrile,
2-(1-ethylpropyloxyimino)propanedinitrile,
2-(1,1-dimethylpropyloxyimino)propanedinitrile,
2-(1,2-dimethylpropyloxyimino)propanedinitrile,
2-(2,2-dimethylpropyloxyimino)propanedinitrile,
2-hexyloxyiminopropanedinitrile,
2-(1-methylpentyloxyimino)propanedinitrile,
2-(2-methylpentyloxyimino)propanedinitrile,
2-(3-methylpentyloxyimino)propanedinitrile,
2-(4-methylpentyloxyimino)propanedinitrile,
2-(1,2-dimethylbutyloxyimino)propanedinitrile,
2-(1,3-dimethylbutyloxyimino)propanedinitrile,
2-(1-ethylbutyloxyimino)propanedinitrile,
2-(2-ethylbutyloxyimino)propanedinitrile,
2-(1-methyl-2,2-dimethylpropyloxyimino)propanedinitrile,
2-(1,1-dimethyl-2-methylpropyloxyimino)propanedinitrile,
2-(1-ethyl-1-methylpropyloxyimino)propanedinitrile and the like.

From the viewpoints of utility of the compound and the like,

2-propoxyiminopropanedinitrile, 2-isopropoxyiminopropanedinitrile,
2-butoxyiminopropanedinitrile, 2-sec-butoxyiminopropanedinitrile,
2-isobutoxyiminopropanedinitrile and
2-tert-butoxyiminopropanedinitrile are preferable.

2-propoxyiminopropanedinitrile and 2-isopropoxyiminopropanedinitrile are more preferable.

The form of the compound presented by the general formula (2), which is 2-alkoxyiminopropanedinitrile for use in the step (iii), includes the substance, which is isolated and purified reaction product of the step (ii), the crude product, which is isolated but not purified reaction product of the step (ii) and the reaction mixture of the step (ii) et. al. However, it is not limited to them. Therefore, the above compound presented by the general formula (2), which is 2-alkoxyiminopropanedinitrile may be isolated and then used in the step (iii) or it can be used for the step (iii) without isolation. Additionally, the above compound presented by the general formula (2), which is 2-alkoxyiminopropanedinitrile can be purified and then used in the step (iii) or it can be used for the step (iii) without any purification.

(The yields of the steps (i) and (ii))

The total yield of the steps (i) and (ii) is, for example, usually the range of over 60%, preferably the range of 65 to 100% and more preferably the range of 70 to 100%.

The total yield of the steps (i) and (ii) can be calculated from the moles of 2-alkoxyiminopropanedinitrile of the general formula (2) obtained to those of malononitrile of the formula (1) used as raw material.
i.e., The yield is presented by the following formula.

Yield (%) = 100 x {(the moles of the compound presented by the general formula (2) obtained) / (The moles of malononitrile of the formula (1) used as raw material)}

(Step (iii))

Next, the step (iii) will be described.

The step (iii) is the reaction of a compound presented by the general formula (2):

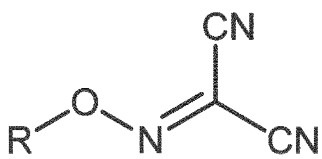

(2)

wherein R is C3 to C6 alkyl group,
with a hydroxylamine compound to produce a compound presented by the general formula (3):

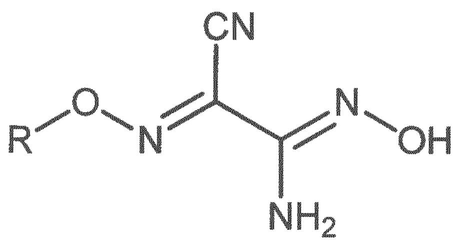

(3)

wherein R is C3 to C6 alkyl group.

(Hydroxylamine compounds)

Examples of hydroxylamine compounds include hydroxylamine and hydroxylamine salts. Examples of the hydroxylamine salts include hydroxylamine sulfate or hydroxylamine hydrochloride. However, they are not limited to these examples. As for hydroxylamine, their aqueous solutions are usually used considering its risk. The concentrations of these aqueous solutions are preferably below 60%. As for the form of hydroxylamine salts, any form is possible as long as the reaction proceeds . The form of hydroxylamine salts includes for example, a solid of hydroxylamine salts only, an aqueous solution or solution of other solvent except water at any concentration and the like. From the viewpoints of price, availability, safety and the like, an aqueous solution is a preferable form of hydroxylamine salts. Additionally,

hydroxylamine compounds can be used alone or as a mixture of two or more compounds at any mixing ratio.

(The amount of hydroxylamine compounds to be used)

The hydroxylamine compounds can be used in any amount as long as the reaction proceeds. From the viewpoints of yield, inhibition of by-product, economic efficiency and the like, they can be usually used, for example, in a range of 0.8 to 2.0 mol, preferably a range of 0.9 to 1.5 mol, more preferably in a range of 0.9 to 1.3 mol and further preferably in a range of 0.9 to 1.2 mol to 1 mol of the compound presented by the formula (2).

(The base of the step (iii))

It is preferable to use a base when a hydroxylamine salt is used as hydroxylamine compound.

The bases which can be used in the step (iii) are for example, inorganic bases such as alkali metal hydroxides, alkali earth metal hydroxides, alkali metal carbonates, alkali earth metal carbonates, alkali metal hydrogen carbonates, alkali earth metal hydrogen carbonates and the like, organic bases such as pyridines, quinolines, isoquinolines, tertiary amines, secondary amines, primary amines, aromatic amines, cyclic amines, alkali metal carboxylates, alkali earth metal carboxylates and the like. However, they are not limited to them.

From the viewpoints of reactivity, yield, price, easy handling and the like, the examples of bases used in the step (iii) are preferably alkali metal hydroxides, alkali metal carbonates, alkali metal hydrogen carbonates, carboxylic acid alkali metal salts, more preferably alkali metal hydroxides, alkali metal carbonates, alkali metal hydrogen carbonates and further preferably alkali metal carbonates, alkali metal hydrogen carbonate. The specific examples of bases used in the step (iii) are preferably sodium hydroxide, potassium hydroxide, sodium carbonate, potassium carbonate, sodium hydrogen carbonate, potassium hydrogen carbonate, sodium acetate, potassium acetate, more preferably sodium hydroxide, potassium hydroxide, sodium carbonate, potassium carbonate, sodium hydrogen carbonate, potassium hydrogen carbonate and further preferably sodium carbonate, potassium carbonate, sodium hydrogen carbonate and potassium hydrogen carbonate.

As for the form of the bases for use in the step (iii), the bases can be used in any form as long as the reaction proceeds. For example, the form includes a solid, a liquid, an aqueous solution or a solution from any other solvents except water at any concentration of a base. Additionally, a base can be used alone or as a mixture of two or more bases at any mixing ratios in the step (iii).

(The amount of base used in the step (iii))

A base can be used in the step (iii) in any amount as long as the reaction proceeds. From the viewpoints of yield, inhibition of by-product, economic efficiency and the like, usually 0.8 to 2.0 mol, preferably 0.9 to 1.5 mol, more preferably 0.9 to 1.3 mol and further preferably 0.9 to 1.2 mol of a base can be used to 1 mol of the compound of formula (2).

(Solvent of the step (iii))

The reaction of the step (iii) may be conducted without any solvent. However, it is preferable to use a solvent from the viewpoints of its smooth proceeding and the like. As long as the reaction of the step (iii) proceeds, any solvent can be used. Examples of solvent for use in the step (iii) include water, ethers, alcohols, nitriles, amides, alkyl ureas, sulfoxides, sulfones, carbonate esters, ketones, carboxylic acid esters, carboxylic acids, nitrated aliphatic hydrocarbons, aromatic heterocycles and the like and mixtures consisting of these solvents at any mixing ratio. However, they are not limited to them.

From the viewpoints of price, easy handling and the like, the solvents for use in the step (iii) are preferably for example, ethers, alcohols, nitriles, amides, alkyl ureas, sulfoxides, sulfones, water and mixtures of these solvents, more preferably ethers, alcohols, nitriles, amides, water and mixtures of these solvents and further preferably alcohols, water and mixtures of these solvents.

The specific examples of solvents for use in the step (iii) include preferably tetrahydrofuran (THF), diisopropyl ether, dibutyl ether, cyclopentyl methyl ether (CPME), methyl-tert-butyl ether, methanol, ethanol, propanol, 2-propanol, butanol, acetonitrile, N,N-dimethyl formamide (DMF), N,N-dimethyl acetamide (DMAC), N-methyl pyrrolidone (NMP), N,N'-dimethyl imidazolidinone (DMI), dimethyl sulfoxide (DMSO), sulfolane, water and mixtures of these solvents, more preferably tetrahydrofuran (THF), diisopropyl ether, dibutyl ether, cyclopentyl methyl ether (CPME), methyl-tert-butyl ether, methanol, ethanol, propanol, 2-propanol, butanol, acetonitrile, N,N-dimethylformamide (DMF), N,N-dimethyl acetamide (DMAC), N-methyl pyrrolidone (NMP), water and mixtures of these solvents, further preferably methanol, ethanol, propanol, 2-propanol, water and mixtures of these solvents and especially preferably the mixture of methanol and water.

(The amount of solvent used in the step (iii))

The solvent can be used in any amount in the step (iii) as long as the reaction system can be stirred thoroughly. From the viewpoints of reactivity, inhibition of by-product, economic efficiency and the like, for example, they can be usually used in a range of 0 to 10.0 L, preferably in a range of 0.01 to 10.0 L, more preferably in a range of 0.1 to 5.0 L and further preferably in a range of 0.2 to 3.0 L to 1 mol of malononitrile presented by the formula (1).

(Reaction temperature of the step (iii))

There is no limitation in reaction temperature of the step (iii) . From the viewpoints of yield, inhibition of by-product, economic efficiency and the like, usually the temperature range of -30° C (minus 30° C) to 100° C, preferably the range of -20° C (minus 20° C) to 70° C, more preferably the range of -10° C (minus 10° C) to 50° C and further preferably the range of -10° C (minus 10° C) to 35° C can be exemplified.

(Reaction time of the step (iii))

There is no limitation in reaction time of the step (iii) . From the viewpoints of yield, inhibition of by-product, economic efficiency and the like, usually the range of 0.5 to 48 hours, preferably the range of 0.5 to 24 hours, more preferably the range of 1 to 12 hours can be exemplified.
(The product of the step (iii);
2-amino-2-hydroxyimino-N-alkoxyacetimidoylcyanide presented by the general formula (3))
The specific examples of

2-amino-2-hydroxyimino-N-alkoxyacetimidoylcyanide presented by the general formula (3) obtained in the step (iii) include
2-amino-2-hydroxyimino-N-propoxyacetimidoylcyanide,
2-amino-2-hydroxyimino-N-isopropoxyacetimidoylcyanide,
2-amino-2-hydroxyimino-N-butoxyacetimidoylcyanide,
2-amino-2-hydroxyimino-N-sec-butoxyacetimidoylcyanide,
2-amino-2-hydroxyimino-N-isobutoxyacetimidoylcyanide,
2-amino-2-hydroxyimino-N-tert-butoxyacetimidoylcyanide,
2-amino-2-hydroxyimino-N-pentyloxyacetimidoylcyanide,
2-amino-2-hydroxyimino-N-(1-methylbutyloxy)acetimidoylcyanide,
2-amino-2-hydroxyimino-N-(2-methylbutyloxy)acetimidoylcyanide,
2-amino-2-hydroxyimino-N-(3-methylbutyloxy)acetimidoylcyanide,
2-amino-2-hydroxyimino-N-(1-ethylpropyloxy)acetimidoylcyanide,
2-amino-2-hydroxyimino-N-(1,1-dimethylpropyloxy)acetimidoylcyanide,
2-amino-2-hydroxyimino-N-(1,2-dimethylpropyloxy)acetimidoylcyanide,
2-amino-2-hydroxyimino-N-(2,2-dimethylpropyloxy)acetimidoylcyanide,
2-amino-2-hydroxyimino-N-hexyloxyacetimidoylcyanide,
2-amino-2-hydroxyimino-N-(1-methylpentyloxy)acetimidoylcyanide,
2-amino-2-hydroxyimino-N-(2-methylpentyloxy)acetimidoylcyanide,
2-amino-2-hydroxyimino-N-(3-methylpentyloxy)acetimidoylcyanide,
2-amino-2-hydroxyimino-N-(4-methylpentyloxy)acetimidoylcyanide,
2-amino-2-hydroxyimino-N-(1,2-dimethylbutyloxy)acetimidoylcyanide,
2-amino-2-hydroxyimino-N-(1,3-dimethylbutyloxy)acetimidoylcyanide,
2-amino-2-hydroxyimino-N-(1-ethylbutyloxy)acetimidoylcyanide,
2-amino-2-hydroxyimino-N-(2-ethylbutyloxy)acetimidoylcyanide,
2-amino-2-hydroxyimino-N-(1-methyl-2,2-dimethylpropyloxy)acetimidoyl cyanide,
2-amino-2-hydroxyimino-N-(1,1-dimethyl-2-methylpropyloxy)acetimidoyl cyanide,
2-amino-2-hydroxyimino-N-(1-ethyl-1-methylpropyloxy)acetimidoylcyani de and the like.

From the viewpoints of utility of the compound and the like,

2-amino-2-hydroxyimino-N-propoxyacetimidoylcyanide,
2-amino-2-hydroxyimino-N-isopropoxyacetimidoylcyanide,
2-amino-2-hydroxyimino-N-butoxyacetimidoylcyanide,
2-amino-2-hydroxyimino-N-sec-butoxyacetimidoylcyanide,

2-amino-2-hydroxyimino-N-isobutoxyacetimidoylcyanide and
2-amino-2-hydroxyimino-N-tert-butoxyacetimidoylcyanide are preferable.
2-Amino-2-hydroxyimino-N-propoxyacetimidoylcyanide and
2-amino-2-hydroxyimino-N-isopropoxyacetimidoylcyanide are more preferable.

(The yield of the step (iii))

The yield of the step (iii) is, for example, usually the range of over 70%, preferably the range of 80 to 100% and more preferably the range of 85 to 100%.

The yield of the step (iii) can be calculated from the moles of 2-amino-2-hydroxyimino-N-alkoxyacetimidoylcyanide of the general formula (3) obtained to those of 2-alkoxyiminopropanedinitrile of the general formula (2) used as raw material.
i.e., The yield is presented by the following formula.

Yield (%) = 100 x {(the moles of the compound of the general formula (3) obtained / (the moles of the compound of the general formula (2) used as raw material)}

Examples

Next, the present invention is described in detail with reference to Examples. However, the present invention is not limited to these Examples.

Herein, room temperature indicates 10 to 35° C.

($^1$H nuclear magnetic resonance spectrum ($^1$H-NMR))

$^1$H nuclear magnetic resonance spectrum analysis ($^1$H-NMR) was conducted by using Oxford NMR300 (Varian Medical System) and tetra-methyl silane as internal standard substance.

(Measuring method of pH)

pH measurement was carried out by using a hydrogen ion concentration indicator of glass electrode type. For example, model HM-20P manufactured by DKK-TOA Corporation can be used as the indicator

(Differential scanning calorimetry)

The differential scanning calorimetric analysis was carried out using model: DSC-60 model (manufactured by SHIMADZU CORPORATION) at a heating rate of 10° C/min in a temperature range of 40 to 400° C. Regarding the differential scanning calorimetry, the following literatures can be referred to, as required.

(a) : The Chemical Society of Japan ed., "4th ed., Jikkenkagaku Koza (4th ed. Experimental Chemistry Course) 4 Netsu, Atsuryoku (Heat, Pressure)" , pp. 57 to 93 (1992), published by Kumao Ebihara, Maruzen Company, Limited
(b) The Chemical Society of Japan ed., "5th ed. , Jikkenkagaku Koza (Experimental Chemistry Course) 6 Ondo·Netsu, Atsuryoku (Temperature· Heat, Pressure)" , pp. 203 to 205 (2005), published by Seishiro Murata, Maruzen Company, Limited

(HPLC analysis method)

Regarding the HPLC analysis method, the following literatures can be referred to, as required.

(a) : The Chemical Society of Japan ed., "Shin Jikkenkagaku Koza (New Experimental Chemistry Course) 9 Bunsekikagaku (Analytical Chemistry) II", pp. 86 to 112 (1977), published by Shingo Iizumi, Maruzen Company, Limited (For example, regarding packing material-mobile phase combinations that can be used in the column, pp. 93 to 96 can be referred to.)
(b) The Chemical Society of Japan ed., "Jikkenkagaku Koza (Experimental Chemistry Course) 20-1 Bunsekikagaku (Analytical Chemistry)", 5th ed., pp. 130 to 151 (2007), published by Seishiro Murata, Maruzen Company, Limited

(For example, regarding the specific usage and conditions of reversed phase chromatography analysis, pp. 135 to 137 can be referred to.)

Example 1

Production of 2-isopropoxyiminopropanedinitrile

In a reaction vessel were placed 100 ml of water, 66 g of malononitrile (1.00 mol) and 6 g of acetic acid (0.10 mol). While the mixture was stirred under ice cooling, 272 ml of 28% aqueous sodium nitrite solution (1.05 mol) was added dropwise thereto. After the completion of the dropwise addition, the mixture was stirred under ice cooling for 2 hours. Then, in another reaction vessel were placed 534 ml of toluene, 184.5 g of isopropyl bromide (1.50 mol) and 32.2 g of tetrabutylammonium bromide (0.10 mol), while being stirred at 60°C, and the reaction mixture obtained above was added dropwise. Then, the mixture was stirred at the same temperature for 5 hours. The resultant reaction mixture was partitioned between toluene and water, and the toluene layer was separated. The obtained toluene solution was analyzed by a HPLC absolute calibration curve method. As a result, the yield of 2-isopropoxyiminopropanedinitrile was 80%, with respect to the theoretical amount calculated from the amount of the malononitrile used. The toluene solution was worked up in the usual manner to obtain 2-isopropoxyiminopropanedinitrile. The obtained 2-isopropoxyiminopropanedinitrile was identified by NMR analysis.

$^1$H-NMR (300MHz, $CDCl_3$) $\delta$ (ppm): 4.85 (sep, J = 6.3 Hz, 1H), 1.43 (d, J = 6.3 Hz, 6H).

Refractive index ($n_D^{20}$): 1.4405

Differential scanning calorimetric measurement; exothermic onset temperature: 225°C, the amount of heat released: 0.6 kJ/g

Example 2

Production of 2-isopropoxyiminopropanedinitrile

In a reaction vessel were placed 100 ml of water, 66 g of malononitrile (1.00 mol) and 6 g of acetic acid (0.10 mol). While the mixture was stirred under ice cooling, 272 ml of 28% aqueous sodium nitrite solution (1.05 mol) was added dropwise thereto. After the completion of the dropwise addition, the mixture was stirred under ice cooling for 2 hours. Then, in another reaction vessel were placed 534 ml of toluene, 184.5 g of isopropyl bromide (1.50 mol) and 32.2 g of tetrabutylammonium bromide (0.10 mol), while being stirred at 70°C, and the reaction mixture obtained above was added dropwise. During the dropwise addition, the pH of the solution was adjusted to about 6 to 7 using 25% aqueous sodium hydroxide solution. Then, the mixture was stirred at the same temperature for 3 hours. The resultant reaction mixture was partitioned between toluene and water, and the toluene layer was separated. The obtained toluene solution was analyzed by a HPLC absolute calibration curve method. As a result, the yield of 2-isopropoxyiminopropanedinitrile was 75%, with respect to the theoretical amount calculated from the amount of the malononitrile used.

Example 3

Production of 2-isopropoxyiminopropanedinitrile

In a reaction vessel were placed 30 ml of water, 6.60 g of malononitrile (100 mmol) and 7.45 g of sodium nitrite (1.05 mmol) . While the mixture was stirred under ice cooling, 17 ml of 6 N (six normal) hydrochloric acid (102 mmol) was slowly added dropwise thereto. After the completion of the dropwise addition, the mixture was stirred under ice cooling for 1 hour. The reaction mixture was extracted with 50 ml of tetrahydrofuran (THF). To the obtained tetrahydrofuran solution 20 ml of water was added, and the pH of the solution was adjusted to about 6 to 7 using 25% aqueous sodium hydroxide solution. Then, 18.70 g of isopropyl bromide (110 mmol) and 3.22 g of tetrabutylammonium bromide (10 mmol) was added thereto, and then the mixture was stirred at 70°C for 2 hours. The resultant reaction mixture was partitioned between tetrahydrofuran and water, and the tetrahydrofuran layer was separated. The obtained tetrahydrofuran solution was analyzed by a HPLC absolute calibration curve method. As a result, the yield of 2-isopropoxyiminopropanedinitrile was 78%, with respect to the theoretical amount calculated from the amount of the malononitrile used.

Example 4

Production of 2-propoxyiminopropanedinitrile

In a reaction vessel were placed 6 ml of water, 1.32 g of malononitrile (20 mmol) and 1.49 g of sodium nitrite (21 mmol). While the mixture was stirred under ice cooling, 3.8 ml of 6 N (six normal) hydrochloric acid (23 mmol) was slowly added dropwise thereto. After the completion of the dropwise addition, the mixture was stirred under ice cooling for 1 hour. The reaction mixture was extracted with 5 ml of tetrahydrofuran (THF) twice. To the obtained tetrahydrofuran solution 4 ml of water was added, and the pH of the solution was adjusted to about 6 to 7 using 25% aqueous sodium hydroxide solution. Then, 2.71 g of propyl bromide (22 mmol) and 645 mg of tetrabutylammonium bromide (2 mmol) was added thereto, and then the mixture was stirred at 85° C for 3 hours. The resultant reaction mixture was partitioned between tetrahydrofuran and water, and the tetrahydrofuran layer was separated. The obtained tetrahydrofuran solution was analyzed by a HPLC absolute calibration curve method. As a result, the yield of 2-propoxyiminopropanedinitrile was 70%, with respect to the theoretical amount calculated from the amount of the malononitrile used. The tetrahydrofuran solution was worked up in the usual manner to obtain 2-propoxyiminopropanedinitrile. The obtained 2-propoxyiminopropanedinitrile was identified by NMR analysis.

$^1$H-NMR (300MHz, $CDCl_3$) $\delta$ (ppm): 4.53 (t, J = 6.8 Hz, 2H), 1.85 (m, 2H), 1.00 (t, J = 6.8Hz, 3H).

Refractive index ($n_D^{20}$) : 1.4470

Example 5

Production of 2-amino-2-hydroxyimino-N-isopropoxyacetimidoylcyanide

In a reaction vessel were placed 6.20 g of 2-isopropoxyiminopropanedinitrile (45 mmol) and 40 ml of methanol. While the mixture was stirred under ice cooling, solution of 3.30 g of hydroxylamine sulfate (20 mmol) and 8 ml of water, and 2.10 g of sodium carbonate (20 mmol) was added thereto. After being stirred under ice cooling for 1 hour, the mixture was stirred under room temperature for 2 hours. Solvent was evaporated under reduced pressure. The resultant residue was dissolved by addition of water and diethylether. The 2 layers were separated, and obtained organic layer was dried by sodium sulfate, then sodium sulfate was removed by filtration. 6.12 g of 2-amino-2-hydroxyimino-N-isopropoxyacetimidoylcyanide was obtained with a yield of 90%, by filtrate was concentrated and obtained residue was dried by reduced pressure.

$^1$H-NMR (300MHz, $CDCl_3$) $\delta$ (ppm) : 8.60 (brs, 1H), 5.03 (brs, 2H), 4.63 (sep, J = 4.7Hz, 1H), 1.38 (d, J = 4.7Hz, 6H).

Melting point: 155 to 157°C

Example 6

Production of 2-amino-2-hydroxyimino-N-propoxyacetimidoylcyanide

In a reaction vessel were placed 738.1 mg of 2-propoxyiminopropanedinitrile (HPLC purity = 93%, 5.0 mmol) and 5 ml of methanol. While the mixture was stirred under ice cooling, solution of 410.4 mg of hydroxylamine sulfate (2.5 mmol) and 1 ml of water, and 265.0 mg of sodium carbonate (2.5 mmol) was added thereto. The mixture was stirred under ice cooling for 2 hours. Solvent was evaporated under reduced pressure. The resultant residue was dissolved by addition of water and diethylether. The 2 layers were separated, and obtained organic layer was dried by sodium sulfate, then sodium sulfate was removed by filtration. 815.4 mg of 2-amino-2-hydroxyimino-N-propoxyacetimidoylcyanide was obtained with a yield of 95%, by filtrate was concentrated under reduced pressure and obtained residue was dried by reduced pressure.

$^1$H-NMR (300MHz, $CDCl_3$) $\delta$ (ppm): 8.37 (brs, 1H), 5.00 (brs, 2H), 4.34 (t, J = 5.1Hz, 2H), 1.79 (m, 2H), 1.00 (t, J = 5.5Hz, 3H). Melting point: 110 to 113° C

Example 7

Differential scanning calorimetry of 2-hydroxyiminopropanedinitrile

2-Hydroxyiminopropanedinitrile is a known compound, and was produced in the usual manner known to the skilled person, to be subjected to the differential scanning calorimetry.

Differential scanning calorimetric measurement; exothermic onset temperature: 147°C, the amount of heat released: 1.6 kJ/g

Example 8

Differential scanning calorimetry of sodium salt of 2-hydroxyiminopropanedinitrile

Sodium salt of 2-hydroxyiminopropanedinitrile is a known compound, and was produced in the usual manner known to the skilled person, to be subjected to the differential scanning calorimetry.

Differential scanning calorimetric measurement; exothermic onset temperature: 222°C, the amount of heat absorbed: -0.9 kJ/g (minus 0.9 kJ/g)

Reference Example 1

Production of 2-propoxyiminopropanedinitrile

Process described in production example 10 of Kokoku (examined patent publication) No. 06-004647

15.18 g of sodium nitrite (220 mmol) was added to solution of 13.21 g of malononitrile (200 mmol) and 2.40 g of acetic acid (40 mmol) in 100 ml of acetonitrile in 60 to 65° C. After mixing for 1 hour in same temperature, 27.06 g of propylbromide (220 mmol) was added thereto at 65°C. After refluxing for 5 hours, the mixture was cooled to room temperature and left overnight. The obtained mixture was poured to mixture of 180 ml of water and 180 ml of diethylether. The organic layer was separated, was washed with water and saturated brine, and dried by sodium sulfate. Solvent was evaporated under reduced pressure. 11.52 g of 2-propoxyiminopropanedicyanide was obtained with a yield of 42%, by obtained residue was purified by distillation under reduced pressure.

As described above, 2-propoxyiminopropanedinitril was obtained by same process of production example 10 of Kokoku (examined patent publication) No. 06-004647. However, Compared to Example 4, the yield was considerably lower.

Industrial Applicability

According to the present invention, an industrially suitable process for the production of a 2-amino-2-hydroxyimino-N-alkoxyacetimidoylcyanide of the general formula (3) is provided. In addition, a 2-amino-2-hydroxyimino-N-alkoxyacetimidoylcyanide of the general formula (3) is useful as an agricultural chemical by itself, and is also useful as an intermediate for the production of another agricultural chemical.

According to the present invention, the target 2-amino-2-hydroxyimino-N-alkoxyacetimidoylcyanide of the general formula (3) can be produced, avoiding the use of a thermally dangerous compound as an intermediate for the production thereof in the prior art. That is, according to the present invention, the target 2-amino-2-hydroxyimino-N-alkoxyacetimidoylcyanide of the general formula (3) can be produced, without using 2-amino-2-hydroxyimino-N-hydroxyacetimidoylcyanide of the formula (6), which is understood to exhibit a highly dangerous exothermic decomposition.

A novel 2-alkoxyiminopropanedinitrile of the general formula (2), which is provided by the present invention, is a far safer intermediate for producing the target 2-amino-2-hydroxyimino-N-alkoxyacetimidoylcyanide of the general formula (3), than the one of the prior art. The other production intermediates in the present invention also significantly safer than the one of the prior art. That is, the process of the present invention dramatically increases the safety compared to the process of prior art. Therefore, the process of the present invention is safely applicable to production on a larger scale, such as in a pilot plant or in an industrial manufacture.

Simultaneously, the present invention provides a novel and industrially satisfactory production process for 2-alkoxyiminopropanedinitrile of the general formula (2), which is a useful intermediate in the present invention.

According to the present invention, a 2-alkoxyiminopropanedinitrile of the general formula (2) can be produced, without limiting an alkylating agent to the one having high reactivity, that is, regardless of species of alkylating agents, without using an expensive silver salt, with a high yield, when malononitrile is reacted with an alkali metal salt of nitrous acid and then the resulting compound is reacted with an alkylating agent. Furthermore, according to the present invention, a 2-alkoxyiminopropanedinitrile of the general formula (2) can be produced, using recyclable and inexpensive solvent (for example, an organic solvent such as ethers or particularly aromatic hydrocarbon derivatives and the like).

Furthermore, the process of the present invention has a high yield, and can be efficiently implemented on an industrial scale, by a simple operation, under mild conditions, and without using a special reaction apparatus. Additionally, in the process of the present invention, it is considered that amounts of waste are small and properties of the waste are preferable.

Therefore, the present invention is also environmentally friendly, and has high industrial use value.

## Claims

1. A compound of the general formula (2):

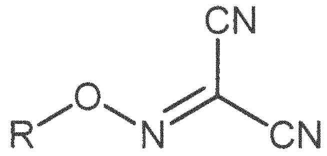

(2)

wherein R is C3 to C6 alkyl group.

2. The compound according to claim 1, wherein R is isopropyl or propyl.

3. A process for the production of a compound of the general formula (3) :

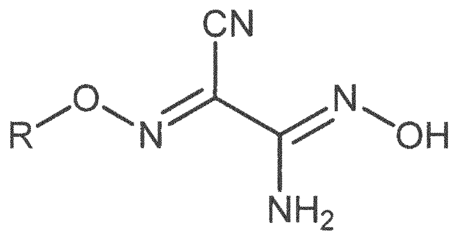

(3)

wherein R is C3 to C6 alkyl group,
which comprises reacting a compound of the general formula (2):

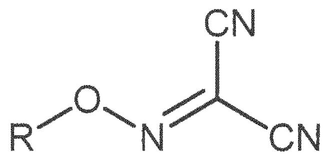

(2)

wherein R is as defined above,
with a hydroxylamine compound.

4. A process for the production of a compound of the general formula (3) :

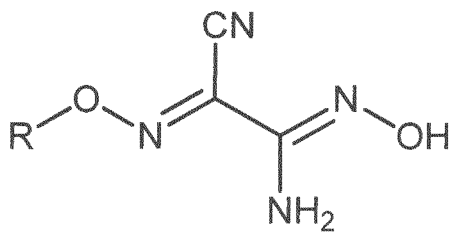

(3)

wherein R is C3 to C6 alkyl group,
which comprises the following steps;

(i) reacting malononitrile of the formula (1):

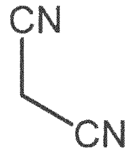

( 1 )

with an alkali metal salt of nitrous acid in the presence of a protic acid;

(ii) reacting the product of step (i) with C3 to C6 alkyl halide in the presence of a phase transfer catalyst to produce a compound of the general formula (2):

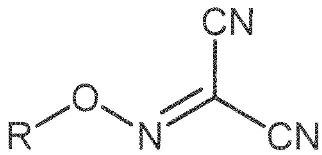

( 2 )

wherein R is as defined above; and

(iii) reacting the resulting compound of the general formula (2) with a hydroxylamine compound.

**5.** The process according to claim 4, wherein the reaction of the step (ii) is carried out in a solvent system consisting of aromatic hydrocarbon derivatives and water or in a solvent system consisting of ethers and water.

**6.** The process according to claim 4, wherein the reaction of the step (ii) is carried out in a solvent system consisting of one or more aromatic hydrocarbon derivatives selected from the group consisting of toluene, xylenes and chlorobenzene, and water.

**7.** The process according to claim 4, wherein the reaction of the step (ii) is carried out in a solvent system consisting of tetrahydrofuran and water.

**8.** The process according to any one of claim 4 to claim 7, wherein R is isopropyl or propyl.

**9.** The process according to any one of claim 4 to claim 7, wherein R is isopropyl.

**10.** The process according to any one of claim 4 to claim 7, wherein R is propyl.

**11.** A process for the production of a compound of the general formula (2) :

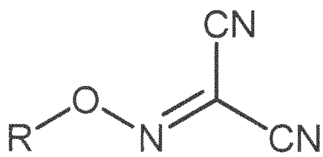

( 2 )

wherein R is C3 to C6 alkyl group,
which comprises the following steps;

(i) reacting malononitrile of the formula (1):

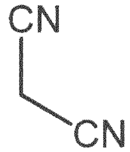

( 1 )

with an alkali metal salt of nitrous acid in the presence of a protic acid; and

(ii) reacting the product of step (i) with C3 to C6 alkyl halide in the presence of a phase transfer catalyst.

**12.** The process according to claim 11, wherein the reaction of the step (ii) is carried out in a solvent system consisting

of aromatic hydrocarbon derivatives and water or in a solvent system consisting of ethers and water.

**13.** The process according to claim 11, wherein the reaction of the step (ii) is carried out in a solvent system consisting of one or more aromatic hydrocarbon derivatives selected from the group consisting of toluene, xylenes and chlorobenzene, and water.

**14.** The process according to claim 11, wherein the reaction of the step (ii) is carried out in a solvent system consisting of tetrahydrofuran and water.

**15.** The process according to any one of claim 11 to claim 14, wherein R is isopropyl or propyl.

**Patentansprüche**

**1.** Verbindung der allgemeinen Formel (2):

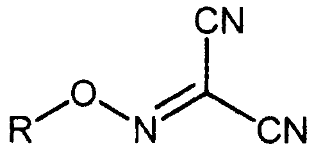

(2)

,

wobei R eine $C_3$- bis $C_6$-Alkylgruppe ist.

**2.** Verbindung nach Anspruch 1, wobei R Isopropyl oder Propyl ist.

**3.** Verfahren zur Herstellung einer Verbindung der allgemeinen Formel (3):

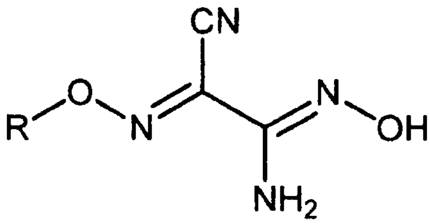

(3)

,

wobei R eine $C_3$- bis $C_6$-Alkylgruppe ist,
welches das Umsetzen einer Verbindung der allgemeinen Formel (2):

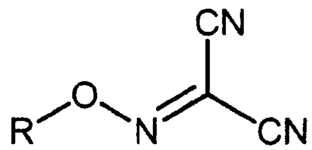

(2)

,

wobei R wie oben definiert ist,
mit einer Hydroxylaminverbindung umfasst.

**4.** Verfahren zur Herstellung einer Verbindung der allgemeinen Formel (3):

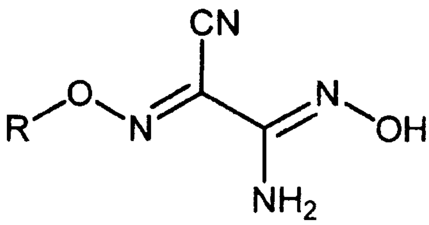

( 3 )

,

wobei R eine $C_3$- bis $C_6$-Alkylgruppe ist, welches die folgenden Schritte umfasst:

(i) das Umsetzen eines Malonsäuredinitrils der Formel (1):

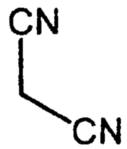

( 1 )

mit einem Alkalimetallsalz von salpetriger Säure in Gegenwart einer protischen Säure;
(ii) das Umsetzen des Produktes aus Schritt (i) mit einem $C_3$- bis $C_6$-Alkylhalogenid in Gegenwart eines Phasentransferkatalysators unter Erhalt einer Verbindung der allgemeinen Formel (2):

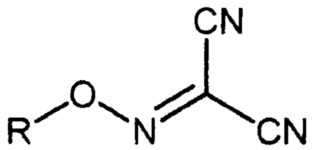

( 2 )

,

wobei R wie oben definiert ist; und
(iii) das Umsetzen der resultierenden Verbindung der allgemeinen Formel (2) mit einer Hydroxylaminverbindung.

**5.** Verfahren nach Anspruch 4, wobei das Umsetzen aus Schritt (ii) in einem Lösungsmittelsystem, bestehend aus aromatischen Kohlenwasserstoffderivaten und Wasser, oder in einem Lösungsmittelsystem, bestehend aus Ethern und Wasser, durchgeführt wird.

**6.** Verfahren nach Anspruch 4, wobei das Umsetzen aus Schritt (ii) in einem Lösungsmittelsystem, bestehend aus einem oder mehreren aromatischen Kohlenwasserstoffderivaten, ausgewählt aus der Gruppe bestehend aus Toluol, Xylolen und Chlorbenzol, und Wasser, durchgeführt wird.

**7.** Verfahren nach Anspruch 4, wobei das Umsetzen aus Schritt (ii) in einem Lösungsmittelsystem, bestehend aus Tetrahydrofuran und Wasser, durchgeführt wird.

**8.** Verfahren nach einem von Anspruch 4 bis Anspruch 7, wobei R Isopropyl oder Propyl ist.

**9.** Verfahren nach einem von Anspruch 4 bis Anspruch 7, wobei R Isopropyl ist.

**10.** Verfahren nach einem von Anspruch 4 bis Anspruch 7, wobei R Propyl ist.

**11.** Verfahren zur Herstellung einer Verbindung der allgemeinen Formel (2):

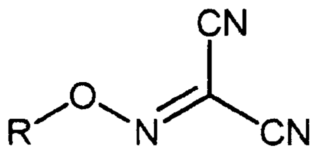

(2)

,

wobei R eine $C_3$- bis $C_6$-Alkylgruppe ist, welches die folgenden Schritte umfasst:

(i) das Umsetzen eines Malonsäuredinitrils der Formel (1):

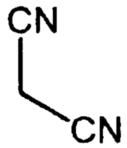

(1)

mit einem Alkalimetallsalz von salpetriger Säure in Gegenwart einer protischen Säure; und
(ii) das Umsetzen des Produktes aus Schritt (i) mit einem $C_3$- bis $C_6$-Alkylhalogenid in Gegenwart eines Phasentransferkatalysators.

**12.** Verfahren nach Anspruch 11, wobei das Umsetzen aus Schritt (ii) in einem Lösungsmittelsystem, bestehend aus aromatischen Kohlenwasserstoffderivaten und Wasser, oder in einem Lösungsmittelsystem, bestehend aus Ethern und Wasser, durchgeführt wird.

**13.** Verfahren nach Anspruch 11, wobei das Umsetzen aus Schritt (ii) in einem Lösungsmittelsystem, bestehend aus einem oder mehreren aromatischen Kohlenwasserstoffderivaten, ausgewählt aus der Gruppe bestehend aus Toluol, Xylolen und Chlorbenzol, und Wasser, durchgeführt wird.

**14.** Verfahren nach Anspruch 11, wobei das Umsetzen aus Schritt (ii) in einem Lösungsmittelsystem, bestehend aus Tetrahydrofuran und Wasser, durchgeführt wird.

**15.** Verfahren nach einem von Anspruch 11 bis Anspruch 14, wobei R Isopropyl oder Propyl ist.

**Revendications**

**1.** Composé de formule générale (2) :

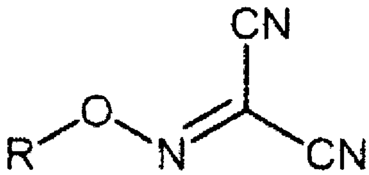

(2)

dans laquelle R est un groupe alkyle en C3 à C6.

**2.** Composé selon la revendication 1, dans lequel R est un isopropyle ou un propyle.

**3.** Procédé de production d'un composé de formule générale (3) :

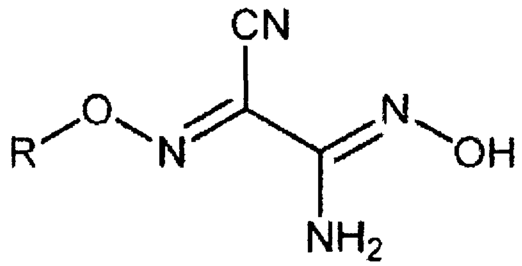

( 3 )

dans laquelle R est un groupe alkyle en C3 à C6, qui comprend la réaction d'un composé de formule générale (2) :

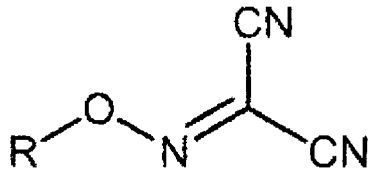

( 2 )

dans laquelle R est tel que défini ci-dessus, avec un composé d'hydroxylamine.

**4.** Procédé de production d'un composé de formule générale (3) :

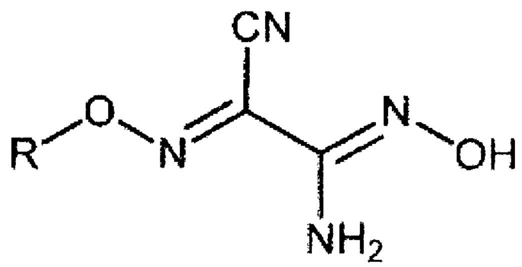

( 3 )

dans laquelle R est un groupe alkyle en C3 à C6, qui comprend les étapes suivantes ;

(i) la réaction du malononitrile de formule (1) :

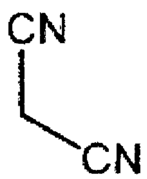

( 1 )

avec un sel de métal alcalin de l'acide nitreux en présence d'un acide protique ;
(ii) la réaction du produit de l'étape (i) avec un halogénure d'alkyle en C3 à C6 en présence d'un catalyseur de transfert de phase afin de produire un composé de formule générale (2) :

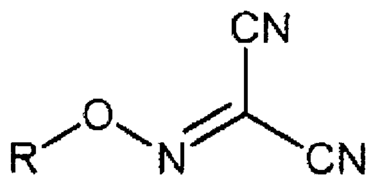

( 2 )

dans laquelle R est tel que défini ci-dessus ; et
(iii) la réaction du composé résultant de formule générale (2) avec un composé d'hydroxylamine.

**5.** Procédé selon la revendication 4, dans lequel la réaction de l'étape (ii) est réalisée dans un système de solvants constitué de dérivés hydrocarbonés aromatiques et d'eau ou dans un système de solvants constitués d'éthers et d'eau.

**6.** Procédé selon la revendication 4, dans lequel la réaction de l'étape (ii) est réalisée dans un système de solvants constitué d'un ou de plusieurs dérivés hydrocarbonés aromatiques choisis dans le groupe constitué du toluène, du xylène et du chlorobenzène, et d'eau.

**7.** Procédé selon la revendication 4, dans lequel la réaction de l'étape (ii) est réalisée dans un système de solvants constitué du tétrahydrofuranne et d'eau.

**8.** Procédé selon l'une quelconque des revendications 4 à 7, dans lequel R est un isopropyle ou un propyle.

**9.** Procédé selon l'une quelconque des revendications 4 à 7, dans lequel R est un isopropyle.

**10.** Procédé selon l'une quelconque des revendications 4 à 7, dans lequel R est un propyle.

**11.** Procédé de production d'un composé de formule générale (2) :

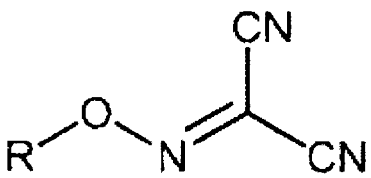
( 2 )

dans laquelle R est un groupe alkyle en C3 à C6,
qui comprend les étapes suivantes ;

(i) la réaction du malononitrile de formule (1) :

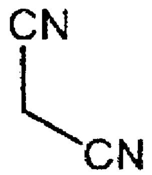
( 1 )

avec un sel de métal alcalin de l'acide nitreux en présence d'un acide protique ; et
(ii) la réaction du produit de l'étape (i) avec un halogénure d'alkyle en C3 à C6 en présence d'un catalyseur de transfert de phase.

**12.** Procédé selon la revendication 11, dans lequel la réaction de l'étape (ii) est réalisée dans un système de solvants constitué de dérivés hydrocarbonés aromatiques et d'eau ou dans un système de solvants constitués d'éthers et d'eau.

**13.** Procédé selon la revendication 11, dans lequel la réaction de l'étape (ii) est réalisée dans un système de solvants constitué d'un ou de plusieurs dérivés hydrocarbonés aromatiques choisis dans le groupe constitué du toluène, du xylène et du chlorobenzène, et d'eau.

**14.** Procédé selon la revendication 11, dans lequel la réaction de l'étape (ii) est réalisée dans un système de solvants constitué du tétrahydrofuranne et d'eau.

**15.** Procédé selon l'une quelconque des revendications 11 à 14, dans lequel R est un isopropyle ou un propyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013080479 A1
- WO 2011161945 A1
- US 20060258719 A1
- JP 4187667 A
- EP 0397511 B1
- JP 6004647 A
- JP 6004647 B
- EP 0069872 B1
- EP 0517041 A1
- EP 0150609 A2
- JP H04187667 B
- US 3234266 A
- JP H0477477 B
- JP S57158769 A
- WO 2013080479 A
- WO 06004647 A

### Non-patent literature cited in the description

- *J. Org. Chem.,* 2000, vol. 65, 1139-1143
- *Bull. Chem. Soc. Jpn.,* 2003, vol. 76, 1063-1070
- Heat, Pressure. Experimental Chemistry Course. Kumao Ebihara, Maruzen Company, Limited, 1992, 57-93
- Temperature· Heat, Pressure. Jikkenkagaku Koza (Experimental Chemistry Course). Seishiro Murata, Maruzen Company, Limited, 2005, 203-205
- Shin Jikkenkagaku Koza (New Experimental Chemistry Course). Analytical Chemistry. Shingo Iizumi, Maruzen Company, Limited, 1977, vol. II, 86-112
- Analytical Chemistry. Jikkenkagaku Koza (Experimental Chemistry Course). Seishiro Murata, Maruzen Company, Limited, 2007, 130-151